# EUROPÄISCHE PATENTANMELDUNG

(11) **EP 4 649 987 A1**
(43) Veröffentlichungstag der Anmeldung: **19.11.2025**
(21) Anmeldenummer: 24176560.1
(22) Anmeldetag: 17.05.2024
(51) Int. Cl.: A61M 25/00

(54) **MEDIZINISCHES ABGABESYSTEM MIT SELBSTSCHLIESSENDEN SCHLITZVENTILEN**

(71) Anmelder: Heraeus Medical GmbH, 61273 Wehrheim (DE)
(72) Erfinder: Vogt, Sebastian, 61273 Wehrheim (DE); Kluge, Thomas, 61273 Wehrheim (DE); Warmbier, Moritz, 61273 Wehrheim (DE); Stangl, Roland, 85368 Moosburg (DE)
(74) Vertreter: Heraeus IP

(57) **Zusammenfassung**

Die Erfindung betrifft eine Vorrichtung zur Abgabe eines medizinischen Fluids an einen Patienten, aufweisend
einen Schlauch, welcher ein erstes medizinisch verträgliches Material mit einer Shore-A-Härte im Bereich von 75 bis 95 aufweist,
wobei der Schlauch einen Innendurchmesser (ID) und einen Außendurchmesser (AD) aufweist,
wobei das Verhältnis von [ID : AD] im Bereich von [1 : 1,8] bis [1 : 2,5] liegt;
wobei der Schlauch ein erstes Ende zur Aufnahme eines Fluids in den Schlauch aufweist;
wobei der Schlauch ein zweites Ende aufweist, das eingerichtet ist, ein Fluid in dem Schlauch zurückzuhalten;
ein oder mehrere Schlitze zur Abgabe eines Fluids aus dem Schlauch, wobei die Schlitze jeweils einen Durchgang bilden, der sich von einer Innenseite des Schlauchs zu einer Außenseite des Schlauchs erstreckt;
und wobei die Schlitze eingerichtet sind, sich abhängig vom Druck eines Fluids innerhalb des Schlauchs reversibel zu öffnen, so dass das Fluid aus den Schlitzen abgegeben wird.

## Beschreibung

Die vorliegende Anmeldung betrifft eine Vorrichtung zur temporären, lokalen Applikation von medizinischen Fluiden über einen Zeitraum von Stunden bis zu mehreren Tagen. Die erfindungsgemäße Vorrichtung kann je nach anatomischer Situation des Implantationsortes hinsichtlich ihrer Länge durch einfaches mechanisches Kürzen angepasst werden, ohne dass ein Funktionsverlust eintritt.

Die lokale Applikation von pharmazeutischen Wirkstoffen, insbesondere von Antibiotika, ist grundsätzlich bekannt und bewährt sich insbesondere bei der Behandlung beziehungsweise der Beruhigung von Infektionen des Knochengewebes. Dabei kann man in nicht-resorbierbare und in resorbierbare beziehungsweise biodegradierbare Wirkstoffträger unterscheiden. Exemplarisch für die nichtresorbierbaren Wirkstoffträger sind die seit 1977 unter dem Markennamen Septopal^{®} bekannten Kugelketten. Diese bestehen aus Polymethylmethacrylat-Kugeln, die das Breitbandantibiotikum Gentamicinsulfat enthalten, wobei diese Kugeln auf Stahlzwirn kettenförmig angeordnet sind. Dieser kettenförmige Wirkstoffträger hat sich seit Jahrzehnten bei der lokalen antibiotischen Behandlung der Osteomyelitis bewährt. Vorteilhaft ist dabei, dass das Gentamcinsulfat in größeren Mengen über einen Zeitraum von mehreren Tagen aus dem Wirkstoffträger freigesetzt wird. Vorteilhaft ist es weiterhin, dass der kettenförmige Wirkstoffträger vom medizinischen Anwender problemlos durch einfaches Abschneiden überzähliger Kugeln an die anatomische Situation am Implantationsort angepasst werden kann. Nachteilig ist, dass der Wirkstoffträger ausschließlich Gentamicinsulfat enthält und dass der medizinische Anwender den Wirkstoffträger nicht mit weiteren Antibiotika, entsprechend der Empfindlichkeit der mikrobiellen Keime, modifizieren kann. Dadurch ist insbesondere die erfolgreiche lokale Behandlung von Infektionen mit Problemkeimen, wie MRSA und VRSA, nur bedingt oder nicht möglich. Die Entfernung der Septopal^{®}-Ketten nach erfolgter Wirkstofffreisetzung ist durch Verwachsung mit Bindegewebe für den Patienten mit einer erheblichen Belastung verbunden.

Exemplarisch für resorbierbare beziehungsweise biodegradierbare Wirkstoffträger sind Vliese und Schwämme aus Kollagen oder Gelatine. Exemplarisch sind dafür die Patente DE3429038, DE3334595, DE2843963. DE3203957 und DE3334595. Diese enthalten Gentamicinsulfat oder Gemische aus Gentamicinsulfat und einem in Wasser gering löslichen Gentamcinsalz. Weiterhin gibt es eine Vielzahl von resorbierbaren beziehungsweise biodegradierbaren Wirkstoffträgern auf Basis von Tricalciumphosphat, Hydroxylapatit, Gips und deren Mischungen sowie auch Kompositmaterialien aus diesen Salzen und organischen Bindemitteln.

Nachteilig an den aufgeführten nicht-resorbierbaren und auch den resorbierbaren beziehungsweise biodegradierbaren Wirkstoffträgern ist, dass der antimikrobielle Wirkstoff durch die gewählte Zusammensetzung festgelegt ist und zusätzlich, dass nach der Implantation des Wirkstoffträgers der Wirkstoff nicht mehr ausgetauscht oder mit anderen Wirkstoffen ergänzt werden kann. Weiterhin basiert die Wirkstofffreisetzung bei allen bisherigen lokalen Wirkstofffreisetzungssystemen auf dem Prinzip der Diffusion, so dass hohe Wirkstoffmengen nur in den ersten Stunden und Tagen freigesetzt werden. Eine Ausnahme bildet die Verwendung von in Wasser gering löslichen Wirkstoffsalzen, bei denen die Wirkstofffreisetzung vom Löslichkeitsgleichgewicht der Wirkstoffsalze abhängt.

Wünschenswert ist daher ein Wirkstoffträger, der eine lokale Applikation beliebiger pharmazeutischer Wirkstoffe zulässt und wobei der pharmazeutische Wirkstoff jederzeit gegen andere fluide pharmazeutische Wirkstoffe ausgetauscht werden kann. Außerdem ist es wünschenswert, dass die Wirkstoffkonzentration, die unmittelbar am Implantationsort erreicht wird, direkt eingestellt werden kann.

Die EP3795196B1 offenbart eine Vorrichtung zur lokalen Applikation eines medizinischen Fluids, aufweisend einen Schlauch, der flexibel verformbar ist und der eine Schlauchwand aufweist, wobei die Schlauchwand eine radial außen liegende äußere Wandung aus einem ersten Material aufweist, und die Schlauchwand eine radial innen liegende innere Wandung aus einem zweiten Material aufweist, die eine innere Leitung des Schlauchs begrenzt, wobei der Schlauch in einem distalen Teilstück des Schlauchs mehrere Öffnungen in der Schlauchwand aufweist, wobei die mehreren Öffnungen die innere Leitung des Schlauchs mit der Umgebung des Schlauchs verbinden, wobei das distale Teilstück des Schlauchs von einem distalen Ende des Schlauchs begrenzt ist, die Vorrichtung ferner aufweisend ein Verschlusselement, mit dem der Schlauch an dem distalen Ende des Schlauchs flüssigkeitsdicht verschlossen ist oder verschließbar ist, wobei das Verschlusselement manuell in das distale Ende des Schlauchs einsetzbar ist, wobei ein proximales Ende des Schlauchs derart mit einem Behälter für das medizinische Fluid flüssigkeitsdurchlässig verbunden ist oder verbindbar ist, dass das medizinische Fluid aus dem Behälter durch das proximale Ende des Schlauchs in die innere Leitung des Schlauchs drückbar ist und durch die mehreren Öffnungen an die Umgebung des Schlauchs herausdrückbar ist.

Die EP3854437B1 beschreibt eine Vorrichtung zur lokalen Applikation eines medizinischen Fluids aufweisend einen Schlauch, wobei der Schlauch mehrere Öffnungen in seiner Schlauchwand aufweist, die eine innere Leitung des Schlauchs mit der Umgebung des Schlauchs verbinden und wobei der Schlauch an einem distalen Schlauchende des Schlauchs verschlossen ist, wobei die Vorrichtung eine äußere Hülse zum fluiddichten Verschließen eines Teils der mehreren Öffnungen aufweist, wobei die äußere Hülse axial verschiebbar um den Schlauch herum angeordnet ist und wobei die äußere Hülse kürzer ist als der Schlauch, so dass die nicht zum verschlossenen Teil der mehreren Öffnungen gehörenden distalen Öffnungen frei liegen.

### BEVORZUGTE AUSFÜHRUNGSFORMEN

Eine Aufgabe der vorliegenden Erfindung besteht darin, eines oder mehrere der oben geschilderten und weitere Probleme des Stands der Technik zu lösen. Eine Aufgabe der Erfindung besteht darin, eine einfache, kostengünstige Vorrichtung zur lokalen Wirkstofffreisetzung zu liefern. In einigen Ausführungsformen kann ein kostengünstiger homogener Schlauch Verwendung finden und somit der Einsatz eines kostenintensiven, koaxialen aus zwei separaten Polymeren gefertigten Schlauchs vermieden werden. Eine solche Vorrichtung kann eine lokale Applikation von medizinischen Fluiden mit beliebiger Zusammensetzung, zum Beispiel antibiotischen Lösungen, ermöglichen. Ein Teil der Vorrichtung kann dazu nach der Implantation im Patienten und ein zweiter Teil der Vorrichtung außerhalb des Patienten angeordnet werden. Die medizinischen Fluide können in dem außerhalb des Patienten befindlichen Teils der Vorrichtung eingebracht werden und durch die Vorrichtung zum Implantationsort geleitet und dort freigesetzt werden. Die Vorrichtung kann plastisch verformbar sein, um den anatomischen Gegebenheiten am Implantationsort folgen zu können. Die Freisetzung von medizinischen Fluiden kann aus längs an der Vorrichtung angeordneten Öffnungen erfolgen. Die Öffnungen können reversibel verschließbar sein, um ein Einwachsen von Bindegewebe und/oder eine Verstopfung der Öffnungen durch z.B. koaguliertes Blut zu verhindern. Dabei ist es wünschenswert, dass sich die Vorrichtung während des Gebrauchs möglichst nicht oder nur minimal radial ausdehnt. Entzündetes humanes Gewebe ist sehr schmerzempfindlich gegenüber Druckbeanspruchung. Daher ist bevorzugt eine deutliche radiale Ausdehnung der Vorrichtung zu vermeiden, die eine Druckbeanspruchung und damit Schmerzen beim Patienten verursachen würde. Vorteilhaft ist weiterhin, dass es bei mehrmaliger Freisetzung der medizinischen Flüssigkeit durch Druckbeaufschlagung nicht zu einem Aufreißen der Öffnungen oder einer sonstigen irreversiblen Vergrößerung der Öffnungen kommt. Bevorzugt kann die Öffnungscharakteristik der Öffnungen der Vorrichtung konstant gehalten werden, um eine gleichmäßige Freisetzung der pharmazeutischen Flüssigkeit über die gesamte Länge der Vorrichtung auch bei mehrfacher Flüssigkeitsfreisetzung zu gewährleisten. Weiterhin kann die Vorrichtung so beschaffen sein, dass der im Patienten positionierbare Teil der Vorrichtung durch Kürzen der Länge eines Schlauchs an die jeweilige anatomische Situation des Patienten angepasst werden kann, ohne dass die Funktion der Vorrichtung beeinträchtigt wird.

Diese Aufgaben werden gelöst durch die hierin beschriebenen Verfahren, Vorrichtungen, Kits und medizinischen Verwendungen, insbesondere denjenigen, die in den Patentansprüchen beschrieben sind.

Bevorzugte Ausführungsformen der Erfindung werden nachfolgend beschrieben.

Eine erste Ausführungsform der Erfindung ist eine Vorrichtung zur Abgabe eines medizinischen Fluids an einen Patienten, aufweisend
einen Schlauch, welcher ein erstes medizinisch verträgliches Material mit einer Shore-A-Härte im Bereich von 75 bis 95 aufweist,
wobei der Schlauch einen Innendurchmesser (ID) und einen Außendurchmesser (AD) aufweist, wobei das Verhältnis von [ID : AD] im Bereich von [1 : 1,8] bis [1 : 2,5] liegt;
wobei der Schlauch ein erstes Ende zur Aufnahme eines Fluids in den Schlauch aufweist; wobei der Schlauch ein zweites Ende aufweist, das eingerichtet ist, ein Fluid in dem Schlauch zurückzuhalten;
ein oder mehrere Schlitze zur Abgabe eines Fluids aus dem Schlauch, wobei die Schlitze jeweils einen Durchgang bilden, der sich von einer Innenseite des Schlauchs zu einer Außenseite des Schlauchs erstreckt;
und wobei die Schlitze eingerichtet sind, sich abhängig vom Druck eines Fluids innerhalb des Schlauchs reversibel zu öffnen, so dass das Fluid aus den Schlitzen abgegeben wird.

Eine zweite Ausführungsform betrifft eine Vorrichtung gemäß der ersten Ausführungsform, wobei die Vorrichtung ausgebildet und eingerichtet ist, ein medizinisches Fluid ausschließlich über die Schlitze abzugeben, wobei bevorzugt eine gleichzeitige Abgabe des Fluids über mehrere Schlitze an unterschiedlichen Positionen entlang der Längsachse des Schlauchs erfolgt.

Eine dritte Ausführungsform betrifft eine Vorrichtung gemäß der ersten oder zweiten Ausführungsform, wobei die Schlitze ausgebildet und eingerichtet sind, sich oberhalb eines Grenzdrucks eines Fluids innerhalb des Schlauchs zu öffnen, welcher 1 bar (10^5 Pa), bevorzugt 1,4 bar, höher als der Druck außerhalb des Schlauchs beträgt, und sich unterhalb dieses Grenzdrucks fluiddicht zu schließen.

Eine vierte Ausführungsform betrifft eine Vorrichtung gemäß einer der vorangehenden Ausführungsformen, wobei die Schlitze eingerichtet sind, sich durch eine elastische Rückstellkraft des ersten Materials des Schlauchs reversibel zu öffnen und schließen.

Eine fünfte Ausführungsform betrifft eine Vorrichtung gemäß einer der vorangehenden Ausführungsformen, wobei die Schlitze jeweils eine Schlitzlänge L aufweisen, und wobei das Verhältnis der Schlitzlänge (L) zum Innendurchmesser des Schlauchs (ID) [L:ID] im Bereich von [1 : 2,2] bis [1 : 2,9] liegt, und/oder das Verhältnis der Schlitzlänge L zum Außendurchmesser AD des Schlauchs [L : AD] im Bereich von [1 : 3,5] bis [1 : 5,5] liegt.

Eine sechste Ausführungsform betrifft eine Vorrichtung gemäß einer der vorangehenden Ausführungsformen, wobei die Vorrichtung ausgebildet und eingerichtet ist, den Außendurchmesser des Schlauchs AD um weniger als 10% zu verändern, wenn der Druck eines Fluids innerhalb des Schlauchs von 0 auf 1 bar (10^5 Pa) höher als der Druck außerhalb des Schlauchs steigt.

Eine siebte Ausführungsform betrifft eine Vorrichtung gemäß einer der vorangehenden Ausführungsformen, wobei die Vorrichtung ausgebildet und eingerichtet ist, die Länge des Schlauchs um weniger als 10%, bevorzugt weniger als 5%, zu verändern, wenn der Druck eines Fluids innerhalb des Schlauchs von 0 auf 1 bar (10^5 Pa) höher als der Druck außerhalb des Schlauchs steigt.

Eine achte Ausführungsform betrifft eine Vorrichtung gemäß einer der vorangehenden Ausführungsformen, wobei der Schlauch mehrere Schlitze aufweist, die jeweils in einem Abstand (A) an der Außenseite des Schlauchs zueinander angeordnet sind, und jeweils eine Schlitzlänge (L) entlang der Außenseite des Schlauchs aufweisen, wobei das Verhältnis von Abstand (A) zur Schlitzlänge (L) [A : L] mindestens [10 : 1] beträgt.

Eine neunte Ausführungsform betrifft eine Vorrichtung gemäß einer der vorangehenden Ausführungsformen, wobei der Schlauch ein zweites Material aufweist, welches eine höhere Shore-A-Härte als das erste Material aufweist, wobei das zweite Material bevorzugt als koaxial umlaufende Schicht oder als Streifen parallel zu einer Längsachse des Schlauchs angeordnet ist, wobei das zweite Material weiter bevorzugt ein Elastomer aufweist, und wobei weiterhin bevorzugt das zweite Material in das erste Material vollständig eingebettet ist.

Eine zehnte Ausführungsform betrifft eine Vorrichtung gemäß der neunten Ausführungsform, wobei das zweite Material einen Röntgenopaker und/oder einen Farbstoff aufweist, wobei der Röntgenopaker bevorzugt Bariumsulfat oder Wolfram umfasst, und wobei der Farbstoff bevorzugt ein absolutes Emissionsmaximum im Bereich von 490 nm bis 575 nm aufweist.

Eine elfte Ausführungsform betrifft eine Vorrichtung gemäß einer der vorangehenden Ausführungsformen, wobei die Schlitze parallel zu einer Längsachse des Schlauchs angeordnet sind.

Eine zwölfte Ausführungsform betrifft eine Vorrichtung gemäß einer der vorangehenden Ausführungsformen, wobei in einer ersten longitudinalen Position in Richtung der Längsachse des Schlauchs ein erster Schlitz an einer ersten radialen Position angeordnet ist, und in einer zweiten longitudinalen Position in Richtung der Längsachse des Schlauchs ein zweiter Schlitz an einer zweiten radialen Position angeordnet ist, wobei die erste radiale Position einen Winkel von etwa 90° zur zweiten radialen Position bildet.

Eine dreizehnte Ausführungsform betrifft eine Vorrichtung gemäß einer der vorangehenden Ausführungsformen, wobei der Schlauch eine Stützstruktur aus Metall aufweist, wobei die Stützstruktur bevorzugt eine Metallbeschichtung, eine Metallfolie, eine Metallspirale oder einen Metallfaden aufweist, und wobei die Stützstruktur bevorzugt in das erste Material eingebettet oder an der Innenseite des Schlauchs angeordnet ist.

Eine vierzehnte Ausführungsform betrifft eine Vorrichtung gemäß einer der vorangehenden Ausführungsformen, wobei das erste medizinisch verträgliche Material ein Polymer aufweist, wobei das Polymer bevorzugt Polyetherurethan oder Ethylen-Propylen-dien-Kautschuk aufweist.

Eine fünfzehnte Ausführungsform betrifft eine Vorrichtung gemäß einer der vorangehenden Ausführungsformen, weiterhin aufweisend einen Fluidverbinder, der an dem ersten Ende des Schlauchs angeordnet ist, bevorzugt lösbar angeordnet ist.

Eine sechzehnte Ausführungsform betrifft einer Ausführungsform gemäß der fünfzehnten Ausführungsform, wobei der Fluidverbinder einen Luer-Lock-Anschluss und/oder ein Rückschlagventil aufweist.

Eine siebzehnte Ausführungsform betrifft eine Vorrichtung gemäß einer der vorangehenden Ausführungsformen, wobei das zweite Ende fluiddicht verschlossen oder verschließbar ist, bevorzugt durch Verschweißen, Verschmelzen oder mittels eines Stopfens oder Schraubverschlusses verschlossen oder verschließbar ist.

Eine achtzehnte Ausführungsform betrifft eine Vorrichtung gemäß einer der vorangehenden Ausführungsformen, wobei die Schlitze durch abschnittsweise Auftrennung des Schlauchs herstellbar oder hergestellt sind, ohne dabei Material aus dem Schlauch zu entfernen, beispielsweise durch Stanzen mit einer Klinge.

Eine neunzehnte Ausführungsform betrifft eine Vorrichtung gemäß einer der vorangehenden Ausführungsformen, wobei die Vorrichtung ausgebildet und eingerichtet ist, die Länge des Schlauchs dauerhaft zu verändern, beispielsweise durch Entfernen eines Schlauchabschnitts am zweiten Endes des Schlauchs.

Eine zwanzigste Ausführungsform betrifft eine Vorrichtung gemäß einer der vorangehenden Ausführungsformen, wobei der Schlauch einstückig ausgebildet ist, und wobei die Vorrichtung keinen zweiten Schlauch aufweist.

Eine erste Ausführungsform eines weiteren Aspekts betrifft ein Kit, aufweisend eine Vorrichtung nach einer der vorangehenden Ausführungsformen, und ein Mittel zum Einbringen eines medizinischen Fluids, welches mit dem ersten Ende des Schlauchs verbindbar ist.

Eine zweite Ausführungsform des Kits gemäß der vorangehenden Ausführungsform weist weiterhin ein Mittel zum Verschließen des zweiten Endes des Schlauchs auf.

Eine dritte Ausführungsform betrifft ein Kit gemäß der ersten oder zweiten Ausführungsform, weiterhin aufweisend ein medizinisches Fluid, welches einen Wirkstoff aufweist, wobei der Wirkstoff bevorzugt ausgewählt ist aus der Gruppe bestehend aus einem Antibiotikum, einem Antimykotikum, einem Antitumorwirkstoff, einem osteoinduktiven Wirkstoff, und einem entzündungshemmenden Wirkstoff.

Eine vierte Ausführungsform betrifft ein Kit gemäß einer der vorangehenden Ausführungsformen, weiterhin aufweisend einen Trokar, wobei der Trokar mit dem ersten Ende des Schlauchs verbindbar ist, bevorzugt lösbar verbindbar ist.

Ein weiterer Aspekt betrifft ein medizinisches Therapieverfahren, wobei das Therapieverfahren die Verabreichung eines medizinischen Fluids an einen Patienten mithilfe einer Vorrichtung der vorangehenden Ausführungsformen oder eines Kits einer der vorangehenden Ausführungsformen aufweist.

Ein weiterer Aspekt betrifft einen medizinischen Wirkstoff zur Anwendung bei einer Knochenoperation, wobei der Wirkstoff ausgewählt ist aus der Gruppe bestehend aus einem Antibiotikum, einem Antimykotikum, einem Antitumorwirkstoff, einem osteoinduktiven Wirkstoff, und einem entzündungshemmenden Wirkstoff, wobei das Verfahren das Inkontaktbringen einer Vorrichtung der vorangehenden Ausführungsformen oder eines Kits einer der vorangehenden Ausführungsformen mit einer Operationswunde eines Patienten und die lokale Verabreichung des Wirkstoffs als medizinisches Fluid an die Operationswunde mithilfe der Vorrichtung oder des Kits aufweist.

### KURZE BESCHREIBUNG DER ZEICHNUNGEN

**Figur 1** zeigt einen Ausschnitt des Schlauchs einer erfindungsgemäßen Vorrichtung in einer Querschnittsansicht.
**Figur 2** zeigt eine Ausführungsform einer erfindungsgemäßen Vorrichtung.
**Figur 3** zeigt einen Fluidverbinder, der lösbar mit einem Schlauch der Vorrichtung verbindbar ist.
**Figur 4** zeigt einen Fluidverbinder mit einem Rückschlagventil.
**Figur 5** zeigt das zweite Ende eines Schlauchs einer erfindungsgemäßen Vorrichtung, die mit einem Stopfen verschließbar ist.
**Figur 6** zeigt das zweite Ende eines Schlauchs einer erfindungsgemäßen Vorrichtung, die eine Stützstruktur im Lumen des Schlauchs aufweist.
**Figur 7** zeigt das zweite Ende eines Schlauchs einer erfindungsgemäßen Vorrichtung, die wobei das zweite Ende eine verschmolzene und dadurch verschlossene Schlauchwand aufweist.
**Figur 8** zeigt das zweite Ende eines Schlauchs mit einem in die Schlauchwand eingebetteten zweiten Material.
**Figur 9** zeigt das zweite Ende eines Schlauchs mit einem in die Schlauchwand eingebetteten zweiten Material als Querschnittsansicht, wobei das zweite Material strangförmig in dem Schlauch angeordnet ist.
**Figur 10** zeigt das zweite Ende eines Schlauchs mit einem in die Schlauchwand eingebetteten zweiten Material als Querschnittsansicht, wobei das zweite Material koaxial umlaufend in dem Schlauch angeordnet ist.
**Figur 11** zeigt einen Ausschnitt eines Schlauchs in einer Längsschnittansicht, wobei zwei Schlitze versetzt zueinander angeordnet sind.
**Figur 12** zeigt einen Ausschnitt eines Schlauchs in einer Querschnittsansicht, wobei zwei Schlitze versetzt zueinander angeordnet sind.
**Figur 13** zeigt einen Ausschnitt eines Schlauchs in einer Längsschnittansicht, wobei die Schlitze in dem Schlauch geschlossen sind.
**Figur 14** zeigt einen Ausschnitt eines Schlauchs in einer Längsschnittansicht, wobei die Schlitze in dem Schlauch geöffnet sind.
**Figur 15** zeigt ein Kit, welches eine hierin beschriebene Vorrichtung, ein Mittel zum Einbringen eines medizinischen Fluids und ein Mittel zum Verschließen des zweiten Endes des Schlauchs umfasst.

### AUSFÜHRLICHE BESCHREIBUNG

Zu den hierin beschriebenen Ausführungsformen, deren Elemente ein bestimmtes Merkmal (z.B. ein Material) "aufweisen", "enthalten", oder "umfassen" wird grundsätzlich immer eine weitere Ausführungsform erwogen, in denen das betreffende Element allein aus dem Merkmal besteht, d.h. keine weiteren Bestandteile umfasst. Das Wort "umfassen" oder "umfassend" wird hierin synonym mit dem Wort "enthalten", "enthaltend", "aufweisen" oder "aufweisend" verwendet.

"Operativ verbunden" oder "operativ verbindbar", bedeutet hierin, dass zwei betreffende Elemente eine funktionelle Beziehung zueinander aufweisen. Beispielsweise kann ein erstes Element eingerichtet sein, ein zweites Element durch eine solche operative Verbindung zu steuern oder zu bewegen. Der Begriff "steuern" umfasst hierin auch die Sperrung oder Freigabe einer Funktion, beispielsweise das Ermöglichen oder die Einschränkung der Bewegung oder sonstigen Funktion eines Elements.

Wenn in einer Ausführungsform ein Element mit dem Singular bezeichnet ist, wird ebenfalls eine Ausführungsform erwogen, bei denen mehrere dieser Elemente vorhanden sind. Die Verwendung eines Begriffs für ein Element im Plural umfasst grundsätzlich auch eine Ausführungsform, in welchem nur ein einzelnes entsprechendes Element enthalten ist. Soweit nicht anders angegeben oder aus dem Zusammenhang eindeutig ausgeschlossen, ist es grundsätzlich möglich und wird hiermit eindeutig in Betracht gezogen, dass Merkmale unterschiedlicher Ausführungsformen auch in den anderen hierin beschriebenen Ausführungsformen vorhanden sein können. Ebenso wird grundsätzlich erwogen, dass alle Merkmale, die hierin in Zusammenhang mit einem Verfahren beschrieben werden, auch für die hierin beschriebenen Erzeugnisse, Vorrichtungen, Kits und Verwendungen anwendbar sind, und umgekehrt. Lediglich aus Gründen der knapperen Darstellung werden alle diese erwogenen Kombinationen nicht in allen Fällen explizit aufgeführt. Auch technische Lösungen, die zu den hierin beschriebenen Merkmalen bekanntermaßen gleichwertig sind, sollen grundsätzlich vom Umfang der Erfindung umfasst sein.

Die hierin beschriebenen technischen Normen und Standards, beispielsweise im Zusammenhang mit Testverfahren, beziehen sich jeweils auf die zum Prioritätsdatum der vorliegenden Anmeldung aktuelle Fassung.

Eine Ausführungsform der Erfindung betrifft eine Vorrichtung zur Abgabe eines medizinischen Fluids an einen Patienten, aufweisend
einen Schlauch, wobei der Schlauch ein erstes Ende zur Aufnahme eines Fluids in den Schlauch aufweist;
wobei der Schlauch ein zweites Ende aufweist, das eingerichtet ist, ein Fluid in dem Schlauch zurückzuhalten;
ein oder mehrere Schlitze zur Abgabe eines Fluids aus dem Schlauch, wobei die Schlitze jeweils einen Durchgang bilden, der sich von einer Innenseite des Schlauchs zu einer Außenseite des Schlauchs erstreckt;
und wobei die Schlitze eingerichtet sind, sich abhängig vom Druck eines Fluids innerhalb des Schlauchs reversibel zu öffnen, so dass das Fluid aus den Schlitzen abgegeben wird.

Die Vorrichtung ist bevorzugt zur Abgabe eines medizinischen Fluids an einen Patienten ausgebildet und eingerichtet. Der Begriff "Fluid" beinhaltet hierin wässrige und nichtwässrige Flüssigkeiten, Gase, und Mischungen davon. Ein "medizinisches Fluid" bezeichnet hierin ein Fluid, welches zur medizinischen Anwendung dient, und eine medizinische Wirkung aufweist. Unter dem Begriff "medizinisches Fluid" werden hierin insbesondere wässrige und nichtwässrige Flüssigkeiten verstanden, die gelöste Wirkstoffe, insbesondere pharmazeutische Wirkstoffe, enthalten können, oder selbst eine medizinische Wirkung haben können. Weiterhin fallen unter diesen Begriff auch Gase und Gas-Flüssigkeitsgemische, die eine pharmakologische Wirkung im humanen oder tierischen Organismus entfalten können. In einer Ausführungsform weist das medizinische Fluid einen Wirkstoff auf. In einer Ausführungsform ist der Wirkstoff ausgewählt aus der Gruppe bestehend aus einem Antibiotikum, einem Antimykotikum, einem Antitumorwirkstoff, einem osteoinduktiven Wirkstoff, und einem entzündungshemmenden Wirkstoff.

In einer Ausführungsform ist der Wirkstoff ein Antibiotikum. In einer Ausführungsform ist das Antibiotikum ausgewählt aus der Gruppe bestehend aus Penicillinen, Cephalosporinen, Carbapenemen, Chinolonen, Makroliden, Lincosamiden, Aminoglycosiden und Glycopeptiden. Beispiele für Penicilline sind Amoxicillin und Benzylpenicillin. Beispiele für Cephalosporine sind Ceftriaxon und Cefuroxim. Beispiele für Carbapeneme sind Meropenem und Imipenem. Beispiele für Chinolone sind Ciprofloxacin und Levofloxacin. Beispiele für Makrolide sind Azithromycin und Clarithromycin. Beispiele für Glycopeptide sind Vancomycin und Teicoplanin. Beispiele für Aminoglykoside sind Gentamicin und Tobramycin. Ein Beispiel für ein Aminoglycosid ist Clindamycin.

In einer Ausführungsform ist der Wirkstoff ein Antimykotikum. Beispiele für Antimykotika umfassen Polyene (z.B. Amphotericin B, Nystatin, Natamycin), Azole (z.B. Fluconazol, Voriconazol), Echinocandine (z.B. Caspofungin, Micafungin), und Allylamine (z.B. Terbinafin). In einer Ausführungsform ist der Wirkstoff ein Antitumorwirkstoff (Zytostatikum). Beispiele für Antitumorwirkstoffe (Zytostatika) umfassen alkylierende Substanzen, Antimetabolite, Naturstoffe, Proteinkinase-Inhibitoren und monoklonale Antikörper. Beispiele für alkylierende Substanzen umfassen Cyclophosphamid, Melphalan und Busulfan. Beispiele für Antimetabolite umfassen Methotrexat, 5-Fluorouracil und Gemcitabin. Beispiele für Naturstoffe umfassen Paclitaxel, Doxorubicin und Vincristin. Beispiele für Proteinkinase-Inhibitoren umfassen Imatinib, Gefitinib und Sunitinib. Beispiele für monoklonale Antikörper umfassen Rituximab, Trastuzumab und Bevacizumab.

In einer weiteren Ausführungsform ist der Wirkstoff ein osteoinduktiver Wirkstoff. Beispiele für osteoinduktive Wirkstoffe umfassen Knochenmorphogenetische Proteine (BMPs), Parathormonverwandte Peptide, Anti-Sklerostin-Antikörper und Wachstumsfaktoren. Beispiele für Knochenmorphogenetische Proteine umfassen BMP-2 und BMP-7. Ein Beispiel für ein Parathormon-verwandtes Peptid ist Teriparatid (PTH 1-34). Ein Beispiel für einen Anti-Sklerostin-Antikörper ist Romosozumab. Beispiele für Wachstumsfaktoren umfassen Fibroblasten-Wachstumsfaktoren (FGFs) und Platelet Derived Growth Factor (PDGF).

In einer weiteren Ausführungsform ist der Wirkstoff ein entzündungshemmender Wirkstoff. Beispiele für entzündungshemmende Wirkstoffe umfassen nichtsteroidale Antirheumatika (NSAR), Glukokortikoide, selektive COX-2-Inhibitoren, Biologika (z.B. TNF-α-Inhibitoren) und Januskinase-Inhibitoren. Beispiele für nichtsteroidale Antirheumatika (NSAR) umfassen Ibuprofen, Diclofenac und Naproxen. Beispiele für Glukokortikoide umfassen Prednison, Dexamethason und Hydrocortison. Beispiele für selektive COX-2-Inhibitoren umfassen Celecoxib und Etoricoxib. Beispiele für TNF-α-Inhibitoren umfassen Infliximab, Adalimumab und Etanercept. Beispiele für Januskinase-Inhibitoren umfassen Tofacitinib und Baricitinib.

In einer Ausführungsform ist der Wirkstoff geeignet zur Behandlung einer Knochenerkrankung. In einer Ausführungsform ist der Wirkstoff ausgewählt aus der Gruppe aus Bisphosphonaten (z.B. Alendronat, Zoledronat), Kalzitonin, selektiven Östrogen-Rezeptor-Modulatoren (z.B. Raloxifen) und Strontiumranelat. In einer Ausführungsform umfasst der Wirkstoff Hyaluronsäure oder ein Kortikosteroid (z.B. Betamethason, Triamcinolon). In einer Ausführungsform umfasst der Wirkstoff ein Calciumsalz. Beispiele für geeignete Calciumsalze umfassen Calciumphosphate und Calciumsulfate. Beispiele für Calciumphosphate umfassen Beta-TCP und Hydroxylapatit.

Die Vorrichtung weist einen Schlauch auf, der mit einem medizinischen Fluid befüllbar ist. Der Schlauch ist bevorzugt ausgestaltet und eingerichtet, ein medizinisches Fluid in einem Innenraum des Schlauchs aufzunehmen. Der hohle Innenraum des Schlauchs wird auch als "Lumen" bezeichnet.

Der Schlauch weist ein medizinisch verträgliches Material auf. "Medizinisch verträglich" bezieht sich hierin auf die Eigenschaft eines Materials, keine schädlichen Auswirkungen auf den menschlichen Körper oder andere biologische Systeme zu haben. Dies bedeutet, dass ein Material sicher und ohne unerwünschte Nebenwirkungen in den Körper eingeführt werden kann, ohne die Gesundheit des Patienten zu gefährden.

Der Schlauch weist ein erstes Ende zur Aufnahme eines Fluids in den Schlauch auf.

Eine Ausführungsform betrifft eine Vorrichtung zur Abgabe eines medizinischen Fluids an einen Patienten, aufweisend
einen Schlauch, welcher ein erstes medizinisch verträgliches Material mit einer Shore-A-Härte im Bereich von 75 bis 95 aufweist,
wobei der Schlauch einen Innendurchmesser (ID) und einen Außendurchmesser (AD) aufweist, wobei das Verhältnis von [ID : AD] im Bereich von [1 : 1,8] bis [1 : 2,5] liegt;
wobei der Schlauch ein erstes Ende zur Aufnahme eines Fluids in den Schlauch aufweist; wobei der Schlauch ein zweites Ende aufweist, das eingerichtet ist, ein Fluid in dem Schlauch zurückzuhalten;
ein oder mehrere Schlitze zur Abgabe eines Fluids aus dem Schlauch, wobei die Schlitze jeweils einen Durchgang bilden, der sich von einer Innenseite des Schlauchs zu einer Außenseite des Schlauchs erstreckt;
und wobei die Schlitze eingerichtet sind, sich abhängig vom Druck eines Fluids innerhalb des Schlauchs reversibel zu öffnen, so dass das Fluid aus den Schlitzen abgegeben wird.

Wenn das Verhältnis von Innendurchmesser zu einen Außendurchmesser des Schlauchs [ID : AD] im Bereich von [1 : 1,8] bis [1 : 2,5] liegt, insbesondere bei einem Schlauch, der ein Material mit einer Shore-A-Härte im Bereich von 75 bis 95 aufweist, kann hierdurch eine unerwünschte Dehnung des Schlauchs verringert oder vermieden werden, wenn das Innere des Schlauchs mit einem Fluid gefüllt ist, dessen Druck deutlich höher als der Umgebungsdruck ist. Hierdurch kann auf eine zusätzliche, stabilisierende Schlauchschicht verzichtet werden.

Wenn das Verhältnis von Innendurchmesser zu einen Außendurchmesser des Schlauchs [ID : AD] im Bereich von [1 : 1,8] bis [1 : 2,5] liegt, wird weiterhin ermöglicht, dass bei der Befüllung des Schlauchs mit einem Fluid und der Druckbeaufschlagung des Fluids kein zu großer Kraftaufwand erforderlich ist.

Der Schlauch weist ein erstes Material auf. Das erste Material weist eine Shore-A-Härte im Bereich von 75 bis 95 auf. In einer Ausführungsform weist das erste Material einer Shore-A-Härte von 80 bis 90, beispielsweise ungefähr 85, auf. Die Shore-A-Härte wird nach ASTM D2240 bestimmt.

In einer Ausführungsform weist der Schlauch einen Massenanteil des ersten Materials von mindestens 50 %, bevorzugt mindestens 60 %, 70 %, 80 % oder mindestens 90 % auf. Dieser Massenanteil wird ohne Berücksichtigung von etwaigen lösbar mit dem Schlauch verbundenen Elementen, wie z.B. einem Fluidverbinder oder Stopfen, oder dem Inhalt des Schlauchinnenraums, wie z.B. einem medizinischen Fluid, berechnet. In einer Ausführungsform besteht der Schlauch im Wesentlichen vollständig aus dem ersten Material. Das erste Material ist bevorzugt ein Polymer, insbesondere ein Elastomer. Bevorzugt umfasst das erste Material ein medizinisch verträgliches Elastomer, oder besteht daraus. Beispiele für medizinisch verträgliche Elastomere umfassen Silikonelastomere, thermoplastische Elastomere (TPEs), Polyisopren, Butylkautschuk, Nitrilkautschuk, Ethylen-Propylen-Dien-Monomer (EPDM), Chloropren-Kautschuk, Fluroelastomere, Perfluorelastomere und Polyacrylatelastomere. Beispiele für Silikonelastomere umfassen Polydimethylsiloxan (PDMS) und flüssiger Silikonkautschuk (LSR).

Beispiele für thermoplastische Elastomere (TPEs) umfassen Styrol-Block-Copolymere (SBCs) wie Styrol-Ethylen-Butylen-Styrol (SEBS), thermoplastische Polyurethane (TPU) und thermoplastische Copolyester (TCE). Beispiele für Polyisopren umfassen natürlichen Gummi und synthetischen Polyisopren. Beispiele für Butylkautschuk umfassen Brombutylkautschuk (BIIR) und Chlorbutylkautschuk (CIIR). Ein bevorzugtes Polyurethan ist ein Polyetherurethan. Polyetherurethane sind durch Polyadditionsreaktion eines Polyetherpolyols mit einem Diisocyanat herstellbar.

In einer Ausführungsform weist das erste Material ein thermoplastisches Elastomer auf. In einer Ausführungsform besteht das erste Material aus einem thermoplastischen Elastomer. In einer Ausführungsform weist das erste Material ein Polyetherurethan oder Ethylen-Propylen-dien-Kautschuk auf. In einer Ausführungsform umfasst das erste Material ein Polyetherurethan. In einer Ausführungsform besteht das erste Material aus Polyetherurethan.

In einer Ausführungsform weist das erste Material nur ein einziges Elastomer auf, d. h. dem ersten Material ist kein zweites Elastomer beigemischt. In einer Ausführungsform weist das erste Material mindestens zwei unterschiedliche Materialien, beispielsweise zwei unterschiedliche Elastomere, auf. Hierdurch kann beispielsweise die Härte des ersten Materials auf einen gewünschten Zielwert eingestellt werden. Das erste Material kann ein Copolymer aufweisen. Das Copolymer kann ein thermoplastisches Elastomer sein. Bevorzugt sind Copolymere, welche ein weiches Segment und ein hartes Segment in ihrer Molekülkette aufweisen. Durch das Verhältnis zwischen dem weichen Segment und dem harten Segment innerhalb der Molekülkette des Copolymers können die physikalischen Eigenschaften eines solchen Copolymers eingestellt werden, beispielsweise die Shore-A-Härte des Copolymers. Das harte Segment kann mithilfe eines Verbinders (Linker) mit dem weichen Segment verbunden sein.

Weiterhin kann das erste Material ein Additiv zur Einstellung der Härte aufweisen. Beispiele für solche Additive sind Füllstoffe und Weichmacher. Beispiele für Füllstoffe umfassen Kieselsäuren, Titandioxid, Calciumcarbonat, Bariumsulfat und Kohlenstoff.

Beispiele für Weichmacher umfassen Adipate, Trimellitate, citratbasierte Weichmacher und Ester von mehrwertigen Alkoholen.

Beispielsweise können als Weichmacher TOTM (Tris(2-ethylhexyl)trimellitat), DINCH (Diisononylcyclohexan-1,2-dicarboxylat), ATBC (Acetyltributylcitrat), oder DEHA (Di(2-ethylhexyl)adipat) verwendet werden.

In einer Ausführungsform ist das erste Material frei von Weichmachern. In einer Ausführungsform ist das erste Material frei von Füllstoffen. In einer Ausführungsform ist das erste Material frei von endokrin wirkenden Substanzen wie z.B. der Phtalaten oder Bisphenolen. In einer Ausführungsform kann das erste Material weiterhin ein Gleitmittel aufweisen. Bevorzugt ist das Gleitmittel medizinisch verträglich. Bevorzugt ist das Gleitmittel frei von polyhalogenierten Substanzen und Silikonen. In einer Ausführungsform weist das Gleitmittel einen Naturstoff auf, beispielsweise ein Lipid, ein Triglycerid oder ein Biopolymer.

Das erste Material kann einen Young-Elastizitätsmodul von 1,2 × 10^7 Pa bis 2,1 x × 10^7 Pa aufweisen, beispielsweise 1,3 × 10^7 Pa bis 2,0 x × 10^7 Pa, 1,4 × 10^7 Pa bis 1,9 x × 10^7 Pa, 1,5 × 10^7 Pa bis 1,8 x × 10^7 Pa, oder 1,5 × 10^7 Pa bis 1,7 x × 10^7 Pa. In einer Ausführungsform kann das erste Material einen Young-Elastizitätsmodul von ungefähr 1,6 × 10^7 Pa aufweisen. In einer Ausführungsform kann das erste Material einen Young-Elastizitätsmodul von 2000 bis 2500 psi aufweisen. Letzteres entspricht ungefähr 1,4× 10^7 Pa bis 1,7 × 10^7 Pa.

Der Young-Elastizitätsmodul kann nach ASTM D412 bestimmt werden.

Das erste Material ist bevorzugt mithilfe gängiger Sterilisationsverfahren sterilisierbar, d. h. im Rahmen dieser Verfahren beständig gegenüber UV-Strahlung, Gammastrahlung und Behandlung mit Ethylenoxid.

Das erste Material ist bevorzugt mithilfe fachüblicher Extrusionsverfahren und/oder Spritzgussverfahren formbar.

Der Schlauch weist einen Innendurchmesser (ID) und einen Außendurchmesser (AD) auf. Hierin bezieht sich der Begriff "Innendurchmesser" auf das Maß des geradlinigen Abstands zwischen zwei gegenüberliegenden Innenflächen des Schlauchs. Dieses Maß wird entlang der zentralen Längsachse des Schlauchs ermittelt. Der Innendurchmesser wird im entspannten Zustand des Schlauchs festgestellt, das heißt, wenn keine äußeren Kräfte wie Zug, Druck oder Torsion auf den Schlauch einwirken, die dessen ursprüngliche Form verändern könnten. Die Messung berücksichtigt ausschließlich die lichte Weite des Schlauches.

In entsprechender Weise bezieht sich der Begriff "Außendurchmesser" auf das Maß des geradlinigen Abstands zwischen zwei gegenüberliegenden Außenflächen des Schlauchs. Dieses Maß wird senkrecht zur zentralen Längsachse des Schlauches ermittelt. Der Außendurchmesser wird im entspannten Zustand des Schlauches gemessen.

Bevorzugt weist das Verhältnis von Innendurchmesser zu Außendurchmesser einen Wert im Bereich von [1 : 1,8] bis [1 : 2,5] auf, d.h. der Außendurchmesser hat einen Wert, der das 1,8-fache bis 2,5-fache des Innendurchmessers beträgt. In einigen Ausführungsformen weist das Verhältnis von Innendurchmesser zu Außendurchmesser einen Wert im Bereich von [1 : 1,8] bis [1 : 2,5]; [1 : 1,9] bis [1 : 2,4]; [1 : 2,0] bis [1 : 2,3]; oder [1 : 2,1] bis [1 : 2,2] auf.

Der Schlauch weist ein erstes Ende zur Aufnahme eines Fluids in den Schlauch auf. Dies bedeutet, dass an dem ersten Ende des Schlauchs ein Fluid in den Schlauch eingebracht werden kann. Hierzu kann das erste Ende des Schlauchs einen Fluidverbinder aufweisen. Der Fluidverbinder kann zum Anschluss eines Gefäßes oder einer fluidleitenden Verbindung dienen, um eine Flüssigkeit in den Schlauch aufzunehmen. Ein Beispiel für einen Fluidverbinder ist ein Luer-Lock-Anschluss. Über einen solchen Anschluss kann eine handelsübliche Luer-Lock-Spritze flüssigkeitsdicht und fluidleitend mit dem ersten Ende des Schlauchs verbunden werden. Der Fluidverbinder kann lösbar mit dem Schlauch verbindbar sein. Der Fluidverbinder kann operativ mit dem Schlauch verbindbar sein. Der Fluidverbinder kann hierzu einen im Wesentlichen zylindrischen Stutzen mit einer Verdickung aufweisen, um eine kraftschlüssige Verbindung mit dem Schlauch auszubilden.

In einer Ausführungsform kann der Fluidverbinder ein Rückschlagventil aufweisen. Das Rückschlagventil ist bevorzugt eingerichtet, den Austritt eines Fluids aus dem Schlauch durch den Fluidverbinder zu verhindern. Somit kann durch den Fluidverbinder ein Fluid in den Schlauch eingebracht werden, ohne dass ein Rückfluss des Fluids durch den Fluidverbinder erfolgt.

Der Schlauch weist ein zweites Ende auf. Das zweite Ende ist eingerichtet, ein Fluid in dem Schlauch zurückzuhalten. Dies bedeutet, dass das Lumen des Schlauchs im Bereich des zweiten Endes fluiddicht verschlossen ist oder fluiddicht verschließbar ist. Beispielsweise kann der Schlauch am zweiten Ende durch einen Stopfen oder durch eine verschmolzene oder verklebte Schlauchwand verschlossen sein.

Das Vorhandensein von Schlitzen in der Schlauchwand bleibt hiervon unberührt, d. h. es können im Bereich des zweiten Endes auch Schlitze vorgesehen sein, wie hierin beschrieben. Hierdurch kann erreicht werden, dass der Austritt eines Fluids aus dem Schlauch nur durch die Schlitze in der Schlauchwand erfolgen kann, und die Abgabe eines Fluids druckabhängig steuerbar ist.

Der Schlauch weist ein oder mehrere Schlitze zur Abgabe eines Fluids aus dem Schlauch auf. Die Schlitze bilden jeweils einen Durchgang, der sich von einer Innenseite des Schlauchs zu einer Außenseite des Schlauchs erstreckt. Dies bedeutet, dass die Schlitze eine Durchbrechung der Schlauchwand darstellen. Die Schlitze sind eingerichtet, sich abhängig vom Druck eines Fluids innerhalb des Schlauchs reversibel zu öffnen, so dass das Fluid aus den Schlitzen abgegeben wird. Hierdurch können die Schlitze die Funktion von Ventilen erfüllen, welche sich druckabhängig öffnen und schließen können.

Die Schlitze sind bevorzugt derart ausgestaltet, dass sie Schlitzwände aufweisen, die sich im geschlossenen Zustand berühren, und bevorzugt im geschlossenen Zustand eine gemeinsame Kontaktfläche über den gesamten Bereich der Schlitzwände aufweisen.

Hierdurch kann eine Verstopfung der Schlitze verhindert werden, da sich diese nur dann in einem geöffneten Zustand befinden, wenn gleichzeitig ein Fluid aus dem Schlauch durch die Schlitze herausströmt. Auch ein Einwachsen von Zellen und das Eindringen von Gewebebestandteilen kann so verhindert werden. Aufgrund der erfindungsgemäßen vorteilhaften Ausgestaltung der Vorrichtung kann daher gegebenenfalls auf zusätzliche Beschichtungen, komplexe geometrische Ausgestaltungen und/oder den Zusatz von Gleitmitteln oder Antikoagulanzien (z.B. Heparin) verzichtet werden, die bei nichterfindungsgemäßer Ausgestaltung erforderlich sein können. Weiterhin kann gegebenenfalls auf Spülvorgänge der Vorrichtung im implantierten Zustand verzichtet werden.

Durch eine geeignete Wahl des Schlauchmaterials und/oder der hierin beschriebenen geometrischen Ausgestaltung, insbesondere bezüglich der Wandstärke des Schlauchs und der Länge, Tiefe und/oder Anordnung der Schlitze, kann einerseits die druckabhängige Öffnungscharakteristik der Schlitze bei andererseits gleichzeitiger Stabilität der Vorrichtung erzielt oder verbessert werden. Insbesondere kann eine irreversible Verformung oder ein Aufreißen des Schlauchs im Bereich der Schlitze hierdurch wirksam verhindert werden. Weiterhin kann durch die hierin beschriebenen Ausgestaltungen eine für den medizinischen Anwender gut handhabbare Abgabemenge des Fluids erzielt werden. Zudem kann eine erfindungsgemäße Ausgestaltung des Schlauchs eine Öffnungscharakteristik der Schlitze ermöglichen, die eine Öffnung der Schlitze bei einem Grenzdruck erlaubt, welcher mithilfe einer handelsüblichen Kunststoffspritze manuell bequem aufgebaut werden kann. Dies ist beispielsweise bei einem Überdruck von ungefähr 1 bar der Fall.

In einer Ausführungsform sind die Schlitze innerhalb des ersten Materials angeordnet. In einer Ausführungsform sind die Schlitze derart in dem Schlauch angeordnet, dass ihre Öffnungscharakteristik in Bezug auf den Grenzdruck, bei der sich die Schlitze öffnen, aus den Materialeigenschaften des ersten Materials ergibt. In einer Ausführungsform weist der Schlauch ein erstes Material und ein zweites Material auf, wobei die Öffnungscharakteristik der Schlitze unabhängig von dem zweiten Material ist.

In einer Ausführungsform weist der Schlauch am ersten Ende des Schlauchs einen ersten Schlauchbereich auf, welcher eine homogen geschlossene Wandung ohne Schlitze aufweist. In einer Ausführungsform weist der Schlauch weiterhin am zweiten Ende des Schlauchs einen zweiten Schlauchbereich auf, welcher eine Vielzahl von Schlitzen aufweist. In einer Ausführungsform weist der Schlauch im ersten Schlauchbereich keine Schlitze auf, und weist im zweiten Schlauchbereich eine Vielzahl von Schlitzen auf.

Die erfindungsgemäße Vorrichtung ist in einer Ausführungsform ausgebildet und eingerichtet, ein medizinisches Fluid ausschließlich über die Schlitze abzugeben. Bevorzugt ist die

Vorrichtung ausgebildet und eingerichtet, das Fluid über mehrere Schlitze an unterschiedlichen Positionen entlang der Längsachse des Schlauchs abzugeben. In einer Ausführungsform ist die Vorrichtung ausgebildet und eingerichtet, ein medizinisches Fluid ausschließlich druckabhängig über die Schlitze abzugeben. Weiter bevorzugt ist die Vorrichtung ausgebildet und eingerichtet, durch mehrere Schlitze gleichzeitig jeweils denselben Volumenstrom eines Fluids abzugeben. Mit "Volumenstrom" ist hiermit das Volumen eines Fluids pro Zeiteinheit gemeint, welches durch einen betreffenden Schlitz aus dem Schlauch nach außen abgegeben wird.

In einer Ausführungsform sind die Schlitze ausgebildet und eingerichtet sich oberhalb eines Grenzdrucks eines Fluids innerhalb des Schlauchs zu öffnen, welcher 1 bar (10^5 Pa), höher als der Druck außerhalb des Schlauchs beträgt, und sich unterhalb dieses Grenzdrucks fluiddicht zu schließen. Dies bedeutet, dass die Schlitze geschlossen sind, wenn der Überdruck eines Fluids in dem Schlauch geringer als 1 bar ist, und die Schlitze geöffnet sind, wenn der Überdruck eines Fluids in dem Schlauch 1 bar oder höher ist. Mit "Überdruck" ist hierbei die Differenz zwischen dem atmosphärischen Druck und dem Druck des Fluids in dem Schlauch bezeichnet.

In einer Ausführungsform beträgt dieser Grenzdruck ungefähr 1 bar, ungefähr 1,2 bar, ungefähr 1,3 bar, ungefähr 1,4 bar oder mehr als 1,4 bar.

In einer Ausführungsform sind die Schlitze eingerichtet, sich durch eine elastische Rückstellkraft des ersten Materials des Schlauchs reversibel zu öffnen und schließen. Durch einen Überdruck eines Fluids in dem Schlauch können hierbei die Schlitze geöffnet werden, indem das Material des Schlauchs, insbesondere das erste Material des Schlauchs, durch das Fluid auseinandergedrückt wird.

Die Stabilität der Schlitze und der Grenzdruck, bei denen sich die Schlitze öffnen, können abhängig von den Dimensionen des Schlauchs und der Schlitze sein.

Die Schlitze weisen eine Schlitzlänge L auf. In einer Ausführungsform liegt das Verhältnis der Schlitzlänge L zum Innendurchmesser ID des Schlauchs [L:ID] im Bereich von [1 : 2,2] bis [1 : 2,9]. Dies bedeutet, dass der Innendurchmesser des Schlauchs das 2,2-fache bis 2,9-fache der Schlitzlänge beträgt. Das Verhältnis der Schlitzlänge L zum Innendurchmesser ID des Schlauchs [L:ID] kann beispielsweise im Bereich von [1 : 2,3] bis [1 : 2,8], von [1 : 2,4] bis [1 : 2,7], oder von [1 : 2,5] bis [1 : 2,6] liegen. In einer Ausführungsform beträgt das Verhältnis der Schlitzlänge L zum Innendurchmesser ID des Schlauchs [L:ID] ungefähr [1 : 2,5].

In einer Ausführungsform liegt das Verhältnis der Schlitzlänge L zum Außendurchmesser AD des Schlauchs [L : AD] im Bereich von [1 : 3,5] bis [1 : 5,5]. Dies bedeutet, dass der Außendurchmesser des Schlauchs das 3,5 fache bis 5,5 fache der Schlitzlänge beträgt. Beispielsweise liegt das Verhältnis der Schlitzlänge L zum Außendurchmesser AD des Schlauchs [L : AD] im Bereich von [1 : 3,6] bis [1 : 5,4], [1 : 3,7] bis [1 : 5,3], [1 : 3,8] bis [1 : 5,2], [1 : 3,9] bis [1 : 5,1], [1 : 4,0] bis [1 : 5,0], [1 : 4,1] bis [1 : 4,9], [1 : 4,2] bis [1 : 4,8], [1 : 4,3] bis [1 : 4,7], oder [1 : 4,4] bis [1 : 4,6]. In einer Ausführungsform beträgt das Verhältnis der Schlitzlänge L zum Außendurchmesser AD des Schlauchs ungefähr 4,5.

In einer Ausführungsform ist die Vorrichtung ausgebildet und eingerichtet, den Außendurchmesser des Schlauchs AD um weniger als 10% zu verändern, wenn der Druck eines Fluids innerhalb des Schlauchs von 0 auf 1 bar (10^5 Pa) höher als der Druck außerhalb des Schlauchs steigt. Hierdurch kann eine Ausdehnung des Schlauchs verhindert werden, wenn ein Fluid in dem Schlauch den Grenzdruck erreicht, bei welchem sich die Schlitze öffnen. Durch eine Formstabilität des Schlauchs auch bei erhöhtem Druck des Fluids kann eine Reizung von empfindlichem Gewebe vermieden werden. In einigen Ausführungsformen verändert sich der Außendurchmesser des Schlauchs in entsprechender Weise um weniger als 9 %, 8 %, 7 %, 6 % oder 5 %, wenn der Überdruck des Fluids von 0 auf 1 bar steigt.

In einer Ausführungsform ist die Vorrichtung ausgebildet und eingerichtet, die Länge des Schlauchs um weniger als 10%, bevorzugt weniger als 9 %, 8 %, 7 %, 6 % oder 5%, zu verändern, wenn der Druck eines Fluids innerhalb des Schlauchs von 0 auf 1 bar (10^5 Pa) höher als der Druck außerhalb des Schlauchs steigt.

In einer Ausführungsform weist der Schlauch mehrere Schlitze auf, die jeweils in einem Abstand A an der Außenseite des Schlauchs zueinander angeordnet sind, und jeweils eine Schlitzlänge L entlang der Außenseite des Schlauchs aufweisen, wobei das Verhältnis von Abstand (A) zur Schlitzlänge (L) [A : L] mindestens [10 : 1] beträgt. Dies bedeutet, dass der kürzeste Abstand zwischen zwei Schlitzen mindestens das Zehnfache der Schlitzlänge beträgt. Der Abstand zwischen zwei Schlitzen wird hierbei jeweils ausgehend von den benachbarten Enden der Schlitze bestimmt. Die Bestimmung einer Abstandsmessung zwischen zwei benachbarten Schlitzen erfolgt unter Heranziehung einer Messmethodik, die nicht den zentrischen Abstand zwischen den Mittelpunkten der Schlitze ermittelt, sondern den minimalen Abstand zwischen den äußersten Begrenzungen der Schlitze. Diese Bestimmung impliziert eine Messung, die sich auf den kürzesten physikalisch existenten Abstand zwischen den nahestehenden Grenzflächen der Schlitze konzentriert, unabhängig von Form, Ausrichtung und Position der Mittelpunkte der Schlitze zueinander. Demnach wird der minimale Abstand exakt an der Position bestimmt, an dem die nächstgelegenen Punkte zweier benachbarter Schlitze den geringstmöglichen räumlichen Abstand zueinander aufweisen. In einigen Ausführungsformen beträgt das Verhältnis von Abstand (A) zur Schlitzlänge (L) [A : L] mindestens [15 : 1], [20 : 1], oder [30 : 1].

Hierdurch kann ein Aufreißen der Schlitze in Längsrichtung und/oder eine zu starke radiale Dehnung des Schlauchs verhindert werden.

In einer Ausführungsform weist der Schlauch ein zweites Material auf, welches eine höhere Shore-A-Härte als das erste Material aufweist. Beispielsweise kann die Shore-A-Härte des zweiten Materials einen Wert aufweisen, welcher mindestens das 1,1-fache, 1,2-fache, 1,3-fache, 1,4-fache, 1,5-fache oder 2-fache der Shore-A-Härte des ersten Materials beträgt. Das zweite Material kann ein Elastomer umfassen. Ein Elastomer ist ein Kunststoff, welcher gummielastische Eigenschaften aufweist. Die Shore-A-Härte des zweiten Materials kann, wie oben im Zusammenhang mit dem ersten Material beschrieben, mithilfe von Weichmachern und/oder Füllstoff im eingestellt werden. Demnach ist es möglich, dass sich das erste Material und das zweite Material lediglich durch den Gehalt solcher Weichmacher und/oder Füllstoffe unterscheiden. Das erste Material und das zweite Material können demnach gleiche oder verschiedene Elastomere aufweisen. In einer Ausführungsform weist das erste Material ein erstes Elastomer auf, und das zweite Material weist ein zweites Elastomer auf. In einer Ausführungsform weist das zweite Material kein Elastomer auf, welches in dem ersten Material enthalten ist. In einer Ausführungsform weisen das erste Material und das zweite Material dasselbe Elastomer auf.

Das zweite Material kann ein thermoplastisches Elastomer aufweisen. Das zweite Material kann ein Copolymer aufweisen. Das Copolymer kann ein thermoplastisches Elastomer sein. Bevorzugt sind Copolymere, welche ein weiches Segment und ein hartes Segment in ihrer Molekülkette aufweisen. Durch das Verhältnis zwischen dem weichen Segment und dem harten Segment innerhalb der Molekülkette des Copolymers können die physikalischen Eigenschaften eines solchen Copolymers eingestellt werden. Das harte Segment kann mithilfe eines Verbinders (Linker) mit dem weichen Segment verbunden sein.

Weiterhin kann das erste Material ein Additiv zur Einstellung der Härte aufweisen, wie hierin beschrieben.

In einer Ausführungsform ist das zweite Material frei von Weichmachern. In einer Ausführungsform ist das zweite Material frei von Füllstoffen. In einer Ausführungsform ist das zweite Material frei von endokrin wirkenden Substanzen wie z.B. der Phtalaten oder Bisphenolen.

Ausführungsform sind sowohl das erste Material als auch das zweite Material frei von Weichmachern, Füllstoffen und endokrin wirkenden Substanzen. Andere hierin beschriebene Stoffgruppen wie z.B. Röntgenopaker, Polymere oder Farbstoffe sind hierbei nicht als "Füllstoffe" zu verstehen. Als Füllstoffe werden hierin nur solche Stoffe verstanden, deren primäre Funktion die Einstellung der Härte eines ersten Materials oder zweiten Materials ist, und die nicht einer der anderen hierin beschriebenen Stoffgruppen angehören.

In einer Ausführungsform kann das zweite Material weiterhin ein Gleitmittel aufweisen. Bevorzugt ist das Gleitmittel medizinisch verträglich. Bevorzugt ist das Gleitmittel frei von polyhalogenierten Substanzen und Silikonen.

In einer Ausführungsform weist das erste Material ein Polyetherurethan auf, und das zweite Material weist ein Polyetherblockamid auf.

Das zweite Material kann als koaxial umlaufende Schicht in dem Schlauch bzw. an einer Oberfläche des Schlauchs angeordnet sein. Das zweite Material kann als Streifen parallel zu einer Längsachse des Schlauchs angeordnet sein. Das zweite Material kann an der Innenseite des Schlauchs oder an der Außenseite des Schlauchs angeordnet sein. Das zweite Material kann in das erste Material eingebettet sein, beispielsweise vollständig eingebettet sein. In einer Ausführungsform ist das zweite Material derart in dem Schlauch angeordnet, dass es die Öffnungs- und Schließcharakteristik der Schlitze nicht beeinflusst, insbesondere in Bezug auf den Grenzdruck eines Fluids, bei dem eine Öffnung der Schlitze erfolgt. Das zweite Material kann eingerichtet sein, die Gesamtstruktur des Schlauchs zu stabilisieren, insbesondere auf die longitudinale oder radiale Ausdehnung des Schlauchs bei einer Druckveränderung eines in dem Schlauch aufgenommenen Fluids.

In einer Ausführungsform weist das zweite Material einen Röntgenopaker auf. Beispiele für geeignete Röntgenopaker umfassen Bariumsulfat und Wolfram. In einer Ausführungsform weist das zweite Material einen Farbstoff auf. Der Farbstoff kann ein absolutes Emissionsmaximum im Bereich von 490 nm bis 575 nm aufweisen. Der Farbstoff kann die Sichtbarkeit des Schlauchs verbessern, insbesondere wenn sich der Schlauch im Bereich einer Operationswunde befindet. Farbstoffe mit einer signifikanten Lichtemission im Bereich von 490 nm bis 575 nm können für das menschliche Auge einen besonders guten Kontrast zu rötlich gefärbtem Körpergewebe bilden.

In einer Ausführungsform weist das zweite Material sowohl ein Elastomer, als auch einen Röntgenopaker auf. Der Röntgenopaker kann dabei in das Elastomer eingebettet sein.

In einer Ausführungsform sind die Schlitze parallel zu einer Längsachse des Schlauchs angeordnet. Hierdurch kann eine verbesserte Stabilität der Schlitze und des Schlauchs erreicht werden. In einer Ausführungsform sind mindestens 40 %, mindestens 50 % oder mindestens 90 % aller Schlitze parallel zu einer Längsachse des Schlauchs angeordnet.

Die Schlitze können umlaufend in verschiedene Richtungen weisend angeordnet sein, um ein Fluid aus dem Schlauch in verschiedene Richtungen abzugeben. Hierdurch kann eine räumlich gleichmäßigere Abgabe eines Fluids ermöglicht werden. In einer Ausführungsform ist beispielsweise in einer ersten longitudinalen Position in Richtung der Längsachse des Schlauchs ein erster Schlitz an einer ersten radialen Position angeordnet, und in einer zweiten longitudinalen Position ist in Richtung der Längsachse des Schlauchs ein zweiter Schlitz an einer zweiten radialen Position angeordnet. In einer Ausführungsform bildet hierbei die erste radiale Position einen Winkel von etwa 90° zur zweiten radialen Position. Dies entspricht einer Anordnung, wobei in einer Querschnittsansicht des Schlauchs der erste Schlitz beispielsweise in "12:00-Uhr-Position" und der zweite Schlitz in "9:00-Uhr-Position" oder "3:00-Uhr-Position" angeordnet ist. In einer Ausführungsform beziehen sich die obigen Angaben jeweils auf einen ersten Schlitz und einen zweiten Schlitz in zueinander benachbarten longitudinalen Positionen. In einer Ausführungsform weist der Schlauch paarweise Schlitze auf, die sich jeweils an derselben longitudinalen Position in Richtung der Längsachse des Schlauchs befinden, aber in ihrer radialen Position einen Winkel von 180° zueinander bilden. Dies entspricht einer Anordnung, wobei in einer Querschnittsansicht des Schlauchs der erste Schlitz dieser paarweisen Anordnung beispielsweise in "12:00-Uhr-Position" und der zweite Schlitz dieser paarweisen Anordnung in "6:00-Uhr-Position" angeordnet ist.

In einer Ausführungsform sind die beiden oben genannten Anordnungen gleichzeitig verwirklicht, d. h. ein erstes Schlitzpaar bildet zu einem zweiten Schlitzpaar einen Winkel von etwa 90° bezüglich der radialen Position, während innerhalb des ersten Schlitzpaars und innerhalb des zweiten Schlitzpaars die beiden Schlitze jeweils einen Winkel von 180° zueinander bilden.

In einer Ausführungsform weist der Schlauch eine Stützstruktur aus Metall auf. In einer Ausführungsform weist die Stützstruktur eine Metallbeschichtung, eine Metallfolie, eine Metallspirale oder einen Metallfaden auf. In einer Ausführungsform ist die Stützstruktur in das erste Material eingebettet. In einer Ausführungsform ist die Stützstruktur an der Innenseite des Schlauchs angeordnet. Vergleichbare Stützstrukturen können alternativ oder zusätzlich aus dem hierin beschriebenen zweiten Material gebildet sein.

Um eine gleichmäßige Abgabe eines Fluids aus dem Schlauch zu erzielen, ist es vorteilhaft, das zweite Ende des Schlauchs zu verschließen. Dies bedeutet, dass der Schlauch insbesondere keine Öffnung am zweiten Ende aufweist, welche größer als der maximale Querschnitt der geöffneten Schlitze ist.

Daher ist in einer Ausführungsform der Erfindung das zweite Ende des Schlauchs fluiddicht verschlossen. Hierzu kann der Schlauch beispielsweise am zweiten Ende verschweißt oder verschmolzen sein, sodass der Innenraum des Schlauchs ausschließlich über das erste Ende und die Schlitze eine fluidleitende Verbindung nach außen aufweist.

In einer Ausführungsform ist das zweite Ende des Schlauchs verschließbar. Beispielsweise kann der Schlauch eingerichtet sein, durch einen Stopfen oder Schraubverschluss am zweiten Ende verschlossen zu werden. Ein solcher Verschluss kann der Vorrichtung beiliegen.

In einer Ausführungsform ist der Schlauch am zweiten Ende derart verschlossen oder verschließbar, dass auch bei einem Druck von mindestens 1 bar, bevorzugt mindestens 2 bar, das zweite Ende flüssigkeitsdicht verschlossen ist. Hiervon unberührt bleibt die druckabhängige Öffnung der Schlitze.

Erfindungsgemäß ist der Schlauch eingerichtet, abhängig von dem Druck eines darin aufgenommenen Fluids die Schlitze fluidleitend zu öffnen oder fluiddicht zu verschließen. Eine solche Funktion lässt sich kostengünstig durch eine abschnittsweise Auftrennung des Schlauchs erreichen. Hierbei wird bevorzugt kein Material aus dem Schlauch entfernt, sodass der Schlauch in einer Konfiguration, bei der die Schlitze geschlossen sind, eine glatte und homogene Oberfläche aufweisen kann. Demnach betrifft eine Ausführungsform der Erfindung eine hierin beschriebene Vorrichtung, bei welcher die Schlitze durch abschnittsweise Auftrennung des Schlauchs herstellbar oder hergestellt sind, ohne dabei Material aus dem Schlauch zu entfernen. Dies kann beispielsweise durch Schneiden oder Stanzen mit einer Klinge erfolgen. Infolgedessen können die Schlitze als Einschnitte innerhalb der Wand des Schlauchs ausgestaltet sein. Hierdurch kann eine bessere Gleitfähigkeit, geringere Schädigung von Gewebe, und die Verhinderung eines Verstopfens oder Verklebens der Öffnungen durch Körpergewebe erreicht werden.

Es kann vorteilhaft sein, die Vorrichtung so auszugestalten, dass der medizinische Anwender die Länge des Schlauchs vor dem Inkontaktbringen der Vorrichtung mit einem Patienten kürzen kann. In einer Ausführungsform ist die Vorrichtung daher ausgebildet und eingerichtet, die Länge des Schlauchs dauerhaft zu verändern. Dies kann beispielsweise durch Entfernen eines Schlauchabschnitts am zweiten Endes des Schlauchs geschehen, d. h der Schlauch kann am zweiten Ende abgeschnitten werden, ohne die Funktion der Vorrichtung zu beeinträchtigen. Anschließend kann es, wie zuvor beschrieben, vorteilhaft sein, das zweite Ende des Schlauchs beispielsweise mit einem Stopfen zu verschließen.

Wie oben dargelegt, sind im Stand der Technik vergleichbare Vorrichtungen bekannt, welche zwingend mehrere Schläuche bzw. mehrere gegeneinander verschiebbare Schlauchschichten aufweisen, um die Stabilität der Vorrichtung zu gewährleisten, eine Ausdehnung des gesamten Schlauchs durch ein enthaltenes druckbeaufschlagtes Fluid zu verhindern, und/oder den Bereich der Fluid-Abgabe einzustellen. Die vorliegende Erfindung hingegen ermöglicht eine funktionsfähige Vorrichtung auch bei einer einstückigen Ausgestaltung des Schlauchs, ohne dass es eines weiteren Schlauchs oder einer weiteren, separaten Schlauchschicht bedarf. Eine weitere Ausführungsform betrifft daher eine hierin beschriebene Vorrichtung, bei welcher der Schlauch einstückig ausgebildet ist. In einer Ausführungsform weist Vorrichtung keinen zweiten Schlauch auf. In einer Ausführungsform weist die Vorrichtung keinen zweiten Schlauch und keine zwei gegeneinander verschiebbare Schlauchschichten auf. In einer Ausführungsform bilden das erste Material und das zweite Material gemeinsam eine monolithische Einheit.

Ein weiterer Aspekt der Erfindung betrifft ein Kit, welche eine hierin beschriebene Vorrichtung und ein Mittel zum Einbringen eines medizinischen Fluids umfasst. Das Mittel zum Einbringen eines medizinischen Fluids ist bevorzugt mit dem ersten Ende des Schlauchs verbindbar, bevorzugt operativ verbindbar. Das Mittel zum Einbringen eines medizinischen Fluids kann beispielsweise eine Spritze umfassen. Es können auch weitere fachübliche Vorrichtungen verwendet werden, mit deren Hilfe ein medizinisches Fluid in den Schlauch einbringbar ist. Weitere Beispiele für Mittel zum Einbringen eines medizinischen Fluids umfassen Fluidadapter, Infusionspumpen, und Schwerkraft-Infusionssysteme. Beispiele für Fluidadapter sind Portsysteme und ähnliche Zugänge, sowie Zwischenstücke, die den passenden Anschluss eines Arzneimittelbehälters erlauben. Beispiele für Infusionspumpen umfassen Peristaltikpumpen, Spritzenpumpen, Ballonpumpen, Kolbenpumpen und weitere Pumpen, die im medizinischen Umfeld üblicherweise Gebrauch finden. Das Mittel zum Einbringen eines medizinischen Fluids ist bevorzugt eingerichtet, eine fluiddichte Verbindung mit dem ersten Ende des Schlauchs herzustellen. Beispielsweise können das Mittel zum Einbringen eines medizinischen Fluids und das erste Ende des Schlauchs zueinander passende Elemente einer Luer-Lock-Verbindung aufweisen.

Das Kit kann weiterhin ein Mittel zum Verschließen des zweiten Endes des Schlauchs aufweisen. Beispielsweise kann das Kit einen Stopfen enthalten, mit welchem das zweite Ende des Schlauchs verschließbar ist. In einer Ausführungsform enthält das Kit ein Werkzeug, das zum Verschließen des zweiten Endes des Schlauchs eingerichtet ist. Ein solches Werkzeug kann den Schlauch beispielsweise verpressen, verschmelzen, verklemmen oder mit einem Stopfen versehen, um ihn am zweiten Ende zu verschließen.

Das Kit kann weiterhin ein medizinisches Fluid aufweisen, welches zur Verabreichung mithilfe der Vorrichtung vorgesehen ist. Das Fluid weist bevorzugt einen Wirkstoff auf. In einer Ausführungsform ist der Wirkstoff bevorzugt ausgewählt aus der Gruppe bestehend aus einem Antibiotikum, einem Antimykotikum, einem Antitumorwirkstoff, einem osteoinduktiven Wirkstoff, und einem entzündungshemmenden Wirkstoff. Es können alle der hierin beschriebenen Wirkstoffe sowie weitere in flüssiger Form verabreichbare Wirkstoffe enthalten sein.

In einer weiteren Ausführungsform weist das Kit weiterhin einen Trokar auf. Ein Trokar weist ein spitzes Ende auf, welches die Durchdringung eines Gewebes erlaubt. Weiterhin weist ein Trokar einen Schaft auf, an dem der Trokar geführt werden kann. Der Schaft kann einen zylinderförmigen Hohlraum aufweisen, ähnlich einer Kanüle.

Der Trokar ist bevorzugt mit dem ersten Ende des Schlauchs verbindbar. Bevorzugt ist der Trokar lösbar mit dem ersten Ende des Schlauchs verbindbar. Mithilfe des Trokars kann ein medizinischer Anwender den Schlauch der Vorrichtung in Patientengewebe einführen und hierdurch an einem gewünschten Ort positionieren und fixieren. Ein Trokar kann die schonende und präzise Durchdringung eines Zielgewebes ermöglichen. Hierbei kann ein Kanal zum gewünschten Verabreichungsort geschaffen werden, wodurch sich die Vorrichtung zur Verabreichung eines medizinischen Fluids entsprechend positionieren lässt. Der Schlauch der Vorrichtung kann hierbei durch den mithilfe des Trokars geschaffenen Kanals in ein Patientengewebe eingebracht werden. Bevorzugt ist der Trokar abnehmbar, um nachfolgend an die Positionierung der Vorrichtung mithilfe des Trokars die Aufnahme eines medizinischen Fluids über das erste Ende des Schlauchs zu ermöglichen. Hierbei kann beispielsweise der Trokar vom ersten Ende des Schlauchs entfernt und durch einen Fluidverbinder, beispielsweise einen Luer-Lock-Anschluss, ersetzt werden. Danach kann beispielsweise mithilfe einer Luer-Lock-Spritze ein medizinisches Fluid in den Schlauch gegeben werden, um es mithilfe der Vorrichtung an einem Zielort des Patientengewebes abzugeben.

Ein weiterer Aspekt der Erfindung betrifft ein medizinisches Therapieverfahren, wobei das Therapieverfahren die Verabreichung eines medizinischen Fluids an einen Patienten mithilfe einer hierin beschriebenen Vorrichtung oder eines hierin beschriebenen Kits aufweist.

Das Therapieverfahren kann insbesondere einen, mehrere oder alle der nachfolgend beschriebenen Schritte aufweisen:
- gegebenenfalls Kürzen des Schlauchs am zweiten Ende des Schlauchs
- gegebenenfalls Verschließen des zweiten Endes des Schlauchs
- Verbinden eines Trokars mit dem ersten Ende der Vorrichtung
- Durchdringen eines Patientengewebes mithilfe des Trokars, um einen Zugang zu einem Zielort des Patientengewebes zu schaffen
- Einführen des Schlauchs durch den mithilfe des Trokars geschaffenen Zugang
- Inkontaktbringen des Schlauchs mit einem Zielort des Patientengewebes
- Entfernen des Trokars von dem ersten Ende des Schlauchs
- Verbinden eines Fluidverbinders mit dem ersten Ende des Schlauchs
- Einbringen eines medizinischen Fluids in den Schlauch, bevorzugt über einen Fluidverbinder am ersten Ende des Schlauchs
- Druckbeaufschlagung des medizinischen Fluids innerhalb des Schlauchs, um die Schlitze zu öffnen und das medizinische Fluid durch die Schlitze an das Patientengewebe abzugeben.

In einer Ausführungsform umfasst das Therapieverfahren insbesondere eine Druckbeaufschlagung des medizinischen Fluids innerhalb des Schlauchs, um die Schlitze zu öffnen und das medizinische Fluid durch die Schlitze an das Patientengewebe abzugeben. Dies bedeutet, dass der Druck des medizinischen Fluids innerhalb des Schlauchs auf einen Wert erhöht wird, bei welchem sich die Schlitze aufgrund der gummielastischen Eigenschaften des ersten Materials öffnen.

Ein weiterer Aspekt betrifft einen medizinischen Wirkstoff zur Anwendung bei einer Knochenoperation, wobei der Wirkstoff ausgewählt ist aus der Gruppe bestehend aus einem Antibiotikum, einem Antimykotikum, einem Antitumorwirkstoff, einem osteoinduktiven Wirkstoff, und einem entzündungshemmenden Wirkstoff, wobei das Verfahren das Inkontaktbringen einer hierin beschriebenen Vorrichtung oder eines hierin beschriebenen Kits mit einer Operationswunde eines Patienten und die lokale Verabreichung des Wirkstoffs als medizinisches Fluid an die Operationswunde mithilfe der Vorrichtung oder des Kits aufweist. Beispiele für solche Wirkstoffe sind hierin beschrieben.

Unter "Knochenoperation" im Sinne dieser Patentanmeldung ist jeder chirurgischer Eingriff zu verstehen, der die Behandlung von Knochengewebe des menschlichen oder tierischen Körpers involviert. Dies umfasst, ist aber nicht beschränkt auf, Eingriffe an Knochen selbst, den angrenzenden Weichteilen, Gelenken, sowie assoziierten Strukturen wie Knorpel, Sehnen, Ligamente, Skelettmuskeln und Gefäße. Der Begriff "Knochenoperation" schließt hierbei beispielsweise traumatische Chirurgie, korrektive und konstruktive Chirurgie, orthopädische Chirurgie, Implantationschirurgie, arthroskopische Chirurgie, Wirbelsäulenchirurgie und mikrochirurgische Verfahren ein.

Traumatische Chirurgie befasst sich mit der Behandlung von Frakturen jeglicher Art, Luxationen, knöchernen Verletzungen, Wiederherstellung der Knochenintegrität und Funktion nach akuten sowie chronischen Verletzungen.

Die korrektive und rekonstruktive Chirurgie schließt Eingriffe zur Behebung von Fehlstellungen, Fehlbildungen und Defekten am Knochen ein, die genetischen, traumatischen oder krankheitsbedingten Ursprungs sein können. Dies beinhaltet auch die Rekonstruktion von Knochendefekten nach Tumorresektionen und Infektionsbehandlungen.

Die orthopädische Chirurgie befasst sich mit der Diagnose, Behandlung, Rehabilitation und Prävention von Erkrankungen, Störungen und Verletzungen des Bewegungsapparates.

Die Implantationschirurgie umfasst das Einsetzen von orthopädischen Implantaten, Prothesen, künstlichen Gelenken, Fixierungselementen wie Schrauben, Platten, Nägeln, Drähten und Verankerungssystemen zur Stützung oder Ersetzung von Knochenstrukturen.

Die arthroskopische Chirurgie umfasst minimal-invasive Eingriffe in Gelenken, um Verletzungen oder Erkrankungen wie Arthritis, Meniskusriss oder Kreuzbandläsionen zu behandeln.

Die Wirbelsäulenchirurgie umfasst beispielsweise die Korrektur von Wirbelsäulendeformitäten, der Behandlung von Bandscheibenvorfällen, spinalen Fusionen und dem Stabilisierung von Wirbelsäulenfrakturen.

Mikrochirurgische Verfahren umfassen Eingriffe, welche die Verwendung eines Mikroskops für die präzise Behandlung kleinerer Knochenstrukturen, einschließlich Nervenreparatur und Gefäßanastomosierung, erfordern.

Knochenoperationen umfassen im Zusammenhang mit der vorliegenden Erfindung beispielsweise chirurgische Eingriffe im Bereich von Gelenken, insbesondere Hüftgelenke, Kniegelenke oder Schultergelenke.

Ein weiterer Aspekt betrifft einen medizinischen Wirkstoff zur Therapie oder Prophylaxe einer Knochenerkrankung, wobei der Wirkstoff ausgewählt ist aus der Gruppe bestehend aus einem Antibiotikum, einem Antimykotikum, einem Antitumorwirkstoff, einem osteoinduktiven Wirkstoff, und einem entzündungshemmenden Wirkstoff, wobei das Verfahren die lokale Verabreichung des Wirkstoffs mithilfe einer hierin beschriebenen Vorrichtung oder eines hierin beschriebenen Kits als medizinisches Fluid an ein Zielgewebe eines Patienten umfasst.

Die Knochenerkrankung kann eine Infektion, Verletzung, Degeneration, oder eine entzündliche Erkrankung eines Knochens umfassen. Beispiele für Knochenerkrankungen umfassen Rheuma, Arthritis, Krebserkrankungen, Verletzungen von Knochen oder Knorpelgewebe, Gelenkinfektionen und Osteomyelitis.

### BEISPIELE

Die Erfindung wird nachfolgend anhand von Beispielen weiter verdeutlicht, die jedoch nicht als einschränkend zu verstehen sind. Dem Fachmann wird ersichtlich sein, dass anstelle der hier beschriebenen Merkmale andere äquivalente Mittel in ähnlicher Weise verwendet werden können.

Es wurden folgende Schläuche aus handelsüblichem medizinischem Polyetherurethan mit den Shore-A-Härten 75, 85 und 95 durch Extrusion hergestellt:

| **Schlauchmuster** | **Shore-A-Härte** | **Innendurchmesser [mm]** | **Außendurchmesser [mm]** | **Verhältnis Innendurchmesser zu Außendurchmesser** | |
|---|---|---|---|---|---|
| 1 | 75 | 2,43 | 3,65 | 1 | 1,5 |
| 2 | 75 | 2,03 | 3,65 | 1 | 1,8 |
| 3 | 75 | 1,83 | 3,65 | 1 | 2,0 |
| 4 | 75 | 1,46 | 3,65 | 1 | 2,5 |
| 5 | 75 | 1,21 | 3,65 | 1 | 3,0 |
| 6 | 85 | 2,43 | 3,65 | 1 | 1,5 |
| 7 | 85 | 2,03 | 3,65 | 1 | 1,8 |
| 8 | 85 | 1,83 | 3,65 | 1 | 2,0 |
| 9 | 85 | 1,46 | 3,65 | 1 | 2,5 |
| 10 | 85 | 1,21 | 3,65 | 1 | 3,0 |
| 11 | 95 | 2,43 | 3,65 | 1 | 1,5 |
| 12 | 95 | 2,03 | 3,65 | 1 | 1,8 |
| 13 | 95 | 1,83 | 3,65 | 1 | 2,0 |
| 14 | 95 | 1,46 | 3,65 | 1 | 2,5 |
| 15 | 95 | 1,21 | 3,65 | 1 | 3,0 |

Es wurden drei trapezförmige geschliffene Klingen mit einer Klingenlänge (Länge der Schneidkante parallel zur Schlauchoberfläche) von 0,5 mm, 0,7 mm und 1,0 mm aus Werkzeugstahl hergestellt. Mit diesen wurden die Schlauchmuster in unterschiedlichen Abständen jeweils an einer Stelle des Schlauchs von zwei gegenüberliegenden Seiten geschlitzt. In Vorversuchen mit destilliertem Wasser unter Verwendung einer 5-ml-Spritze zeigte sich, dass mit einer Klingenlänge von 0,7 mm und 1,0 mm eine gleichmäßige Freisetzung von Flüssigkeit erreichbar ist.

Bei einer Klingenlänge von 0,5 mm war die freigesetzte Flüssigkeitsmenge pro Schlitz sehr gering und der Kraftaufwand bei der Betätigung der Spritze unzumutbar hoch. Daher wurde für die entsprechenden Muster keine weitergehende Prüfung durchgeführt.

Weiterhin zeigte sich, dass zur Öffnung der Schlitze bei den Mustern der Beispiele 1,6 und 11 die Handkraft bei der Betätigung der Spritze unangenehm hoch war. Es wurden weiterhin Versuche mit den restlichen Mustern unter Verwendung eines Messschiebers durchgeführt, um die Dehnung der Schlauchmuster mit einer Schlitzlänge von 0,7 mm 1,0 mm zu messen. Es zeigte sich dabei, dass sich bei einem Druck ab 1,0 bar die Schlitze öffneten und die Flüssigkeit aus den Schlitzen austrat. Während des Flüssigkeitsaustritts lag der Druck bei 1,4 bar bis 2,0 bar, wobei dieser Druck manuell mit einer Spritze aufgebaut wurde. In weiteren Versuchen wurde beobachtet, dass bei einer Schlitzlänge von 0,7 mm und einem Abstand zwischen den Schlitzen von kleiner 7 mm, besonders bei einem Abstand von 2 bis 3 mm, temporäre radiale Dehnungen und mitunter Vergrößerungen der Schlitze bei mehrmaliger Druckbeaufschlagung auftraten. In gleicher Weise traten ähnliche Effekte bei einer Schlitzlänge von 1,0 mm und einem Schlitzabstand von weniger als 10 mm auf.

Die folgenden Versuche wurden daher mit einem Schlitzabstand von 7 mm bei 0,7-mm-Schlitzen, und einem Schlitzabstand von 10 mm bei 1,0-mm-Schlitzen durchgeführt. Es wurde jeweils die Dehnung ab dem Öffnen der Schlitze mit einem Messschieber bestimmt. Als Grenzwert wurde eine Dehnung von größer 10% des Außendurchmesser gewählt. Die radiale Dehnung kleiner 10% wurde mit "+" bewertet und eine größere Dehnung als 10% mit "-" bewertet.

| **Schlauchmuster** | **Bewertung** | **Schlitzlänge [mm]** | **Verhältnis** | | |
|---|---|---|---|---|---|
| | | | **Schlitzlänge** | **Innendurchmesser** | **Außendurchmesser** |
| 2 | + | 0,7 | 1,0 | 2,9 | 5,2 |
| 3 | + | 0,7 | 1,0 | 2,6 | 5,2 |
| 4 | + | 0,7 | 1,0 | 2,1 | 5,2 |
| 5 | - | 0,7 | 1,0 | 1,7 | 5,2 |
| 2 | + | 1,0 | 1,0 | 2,0 | 3,6 |
| 3 | + | 1,0 | 1,0 | 1,8 | 3,6 |
| 4 | + | 1,0 | 1,0 | 1,5 | 3,6 |
| 5 | - | 1,0 | 1,0 | 1,2 | 3,6 |
| 7 | + | 0,7 | 1,0 | 2,9 | 5,2 |
| 8 | + | 0,7 | 1,0 | 2,6 | 5,2 |
| 9 | + | 0,7 | 1,0 | 2,1 | 5,2 |
| 10 | - | 0,7 | 1,0 | 1,7 | 5,2 |
| 7 | + | 1,0 | 1,0 | 2,0 | 3,6 |
| 8 | + | 1,0 | 1,0 | 1,8 | 3,6 |
| 9 | + | 1,0 | 1,0 | 1,5 | 3,6 |
| 10 | - | 1,0 | 1,0 | 1,2 | 3,6 |
| 12 | + | 0,7 | 1,0 | 2,9 | 5,2 |
| 13 | + | 0,7 | 1,0 | 2,6 | 5,2 |
| 14 | + | 0,7 | 1,0 | 2,1 | 5,2 |
| 15 | - | 0,7 | 1,0 | 1,7 | 5,2 |
| 12 | + | 1,0 | 1,0 | 2,0 | 3,6 |
| 13 | + | 1,0 | 1,0 | 1,8 | 3,6 |
| 14 | + | 1,0 | 1,0 | 1,5 | 3,6 |
| 15 | - | 1,0 | 1,0 | 1,2 | 3,6 |

### FIGUREN

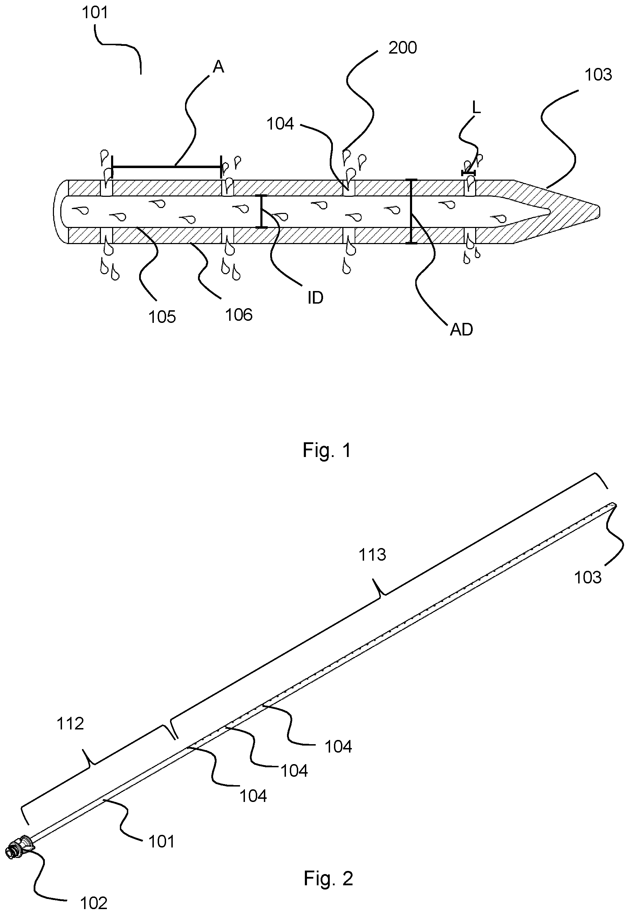

**Figur 1** zeigt einen Ausschnitt des Schlauchs **101** einer erfindungsgemäßen Vorrichtung **100** in einer Querschnittsansicht. Der Schlauch **101** weist ein erstes Ende **102** (in dieser Figur nicht gezeigt) und ein zweites Ende **103** auf. In der Wandung des Schlauchs **101** befinden sich Schlitze **104**, welche sich jeweils von einer Innenseite **105** des Schlauchs zu einer Außenseite **106** des Schlauchs erstrecken. Somit bilden die Schlitze **104** eine fluidleitende Verbindung zwischen der Innenseite **105** und der Außenseite **106** des Schlauchs, durch die ein Fluid **200** aus dem Innenraum des Schlauchs **101** nach außen abgegeben werden kann. Der Schlauch weist einen Innendurchmesser **ID** auf, welcher der lichten Weite des Schlauchs entspricht. Der Schlauch weist weiterhin einen Außendurchmesser **AD** auf, welcher ein Maß des geradlinigen Abstands zwischen zwei gegenüberliegenden Außenflächen des Schlauchs **101** ist. Die Schlitze weisen jeweils eine Schlitzlänge **L** auf. Am zweiten Ende **103** ist der Schlauch **101** verschlossen, sodass das Fluid **200** nur durch die Schlitze **104** austreten kann.

**Figur 2** zeigt eine Ausführungsform einer erfindungsgemäßen Vorrichtung **100.** Die Vorrichtung weist ein erstes Ende **102** auf, welches zur Aufnahme eines Fluids in den Schlauch **101** eingerichtet ist. Der Schlauch weist einen ersten Schlauchbereich **112** auf, welcher eine homogen geschlossene Wandung ohne Schlitze aufweist. Der Schlauch weist weiterhin einen zweiten Schlauchbereich **113** auf, welcher eine Vielzahl von Schlitzen **104** aufweist. Das erste Ende **102** grenzt an den ersten Schlauchbereich **112** an, während das zweite Ende **103** an den zweiten Schlauchbereich **113** angrenzt.

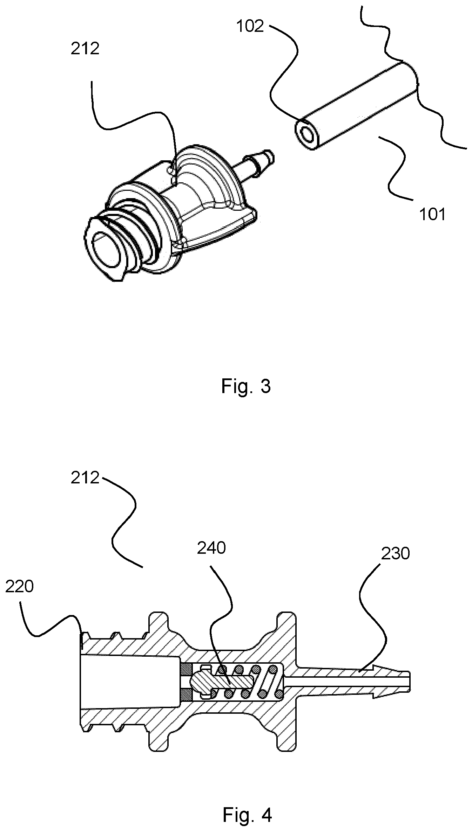

**Figur 3** zeigt einen Fluidverbinder **212**, der lösbar mit einem Schlauch **101** der Vorrichtung verbindbar ist. Der Fluidverbinder ist mit dem ersten Ende **102** des Schlauchs verbindbar, um die Aufnahme eines Fluids in den Schlauch **101** zu ermöglichen.

**Figur 4** zeigt einen Fluidverbinder **212** mit einem Luer-Lock-Anschluss **220**, einem Schlauchverbinder **230**, und einem Rückschlagventil **240.** Der Luer-Lock-Anschluss **220** ermöglicht beispielsweise die lösbare Verbindung der Vorrichtung mit einer Spritze, um ein medizinisches Fluid in die Vorrichtung einzubringen. Gleichzeitig kann die Spritze hierbei der Druckbeaufschlagung des Fluids innerhalb des Schlauchs dienen, um die Schlitze zu öffnen.

Der Schlauchverbinder **230** ermöglicht eine lösbare, fluidleitende und flüssigkeitsdichte Verbindung mit dem ersten Ende **102** des Schlauchs **101.** Der Schlauchverbinder **230** kann hierzu in den Schlauch **101** eingeschoben werden. Der Schlauchverbinder weist einen zylindrischen Stutzen mit einer Verdickung auf, um eine kraftschlüssige, flüssigkeitsdichte Verbindung mit dem Schlauch auszubilden.

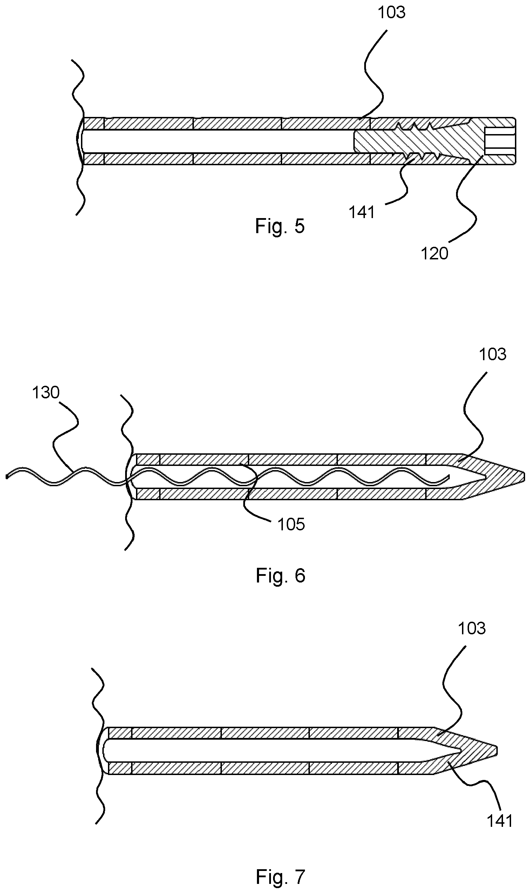

**Figur 5** zeigt das zweite Ende **103** eines Schlauchs einer erfindungsgemäßen Vorrichtung, das mit einem Stopfen **120** verschließbar ist. Der Stopfen **120** ist hier mit einem Gewinde versehen, um eine kraftschlüssige Verbindung mit dem Schlauch herzustellen, so dass der Schlauch am zweiten Ende **103** flüssigkeitsdicht verschließbar ist. Der Stopfen kann in ähnlicher Weise mit umlaufenden Erhebungen versehen sein, die in das flexible erste Material **141** des Schlauchs eingreifen können, um den Schlauch flüssigkeitsdicht zu verschließen.

**Figur 6** zeigt das zweite Ende **103** eines Schlauchs einer erfindungsgemäßen Vorrichtung, die eine Stützstruktur **130** im Lumen des Schlauchs aufweist. Die Stützstruktur ist hier als spiralförmig gewundener Draht aus Metall ausgestaltet, der an der Innenseite **105** des Schlauchs angeordnet ist. Die Stützstruktur **130** kann die Biegefestigkeit des Schlauchs erhöhen, so dass beispielsweise die Bildung von Knicken im Schlauch vermieden werden kann.

**Figur 7** zeigt das zweite Ende eines Schlauchs **103** einer erfindungsgemäßen Vorrichtung, die wobei das zweite Ende **103** eine verschmolzene und dadurch verschlossene Schlauchwand aufweist. Hierbei kann insbesondere das erste Material **141** ein verschmolzenes Schlauchende ausbilden.

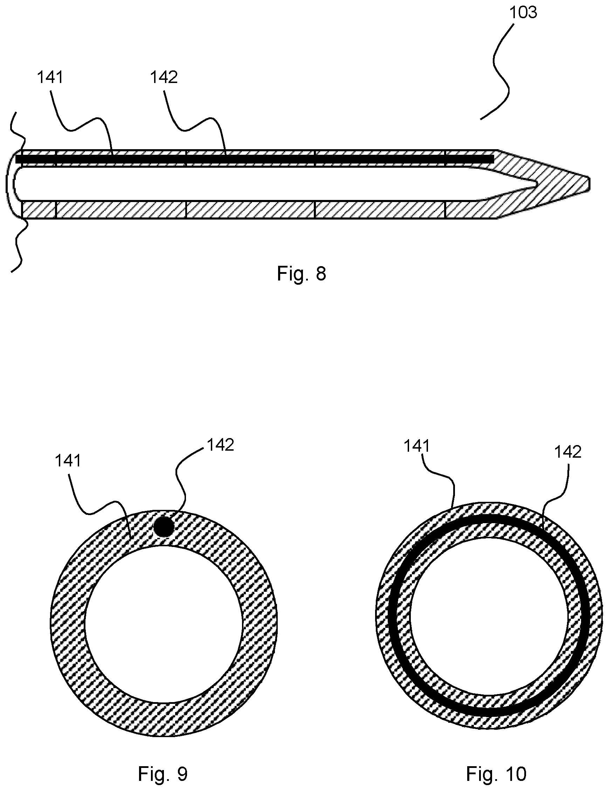

**Figur 8** zeigt das zweite Ende **103** eines Schlauchs mit einem in die Schlauchwand eingebetteten zweiten Material **142.** Das zweite Material **142** weist eine höhere Härte als das umgebende erste Material **141** auf, und erstreckt sich strangförmig parallel zur zentralen Längsachse des Schlauchs in Richtung des zweiten Ende **103** des Schlauchs. Hierdurch kann die Geometrie des Schlauchs stabilisiert werden. Insbesondere kann eine Ausdehnung des Schlauchs aufgrund einer druckbeaufschlagten Flüssigkeit innerhalb des Schlauchs gemindert oder vermieden werden.

**Figur 9** zeigt das zweite Ende eines Schlauchs **103** mit einem in die Schlauchwand eingebetteten zweiten Material **142** als Querschnittsansicht, wobei das zweite Material **142** strangförmig in dem Schlauch angeordnet ist. Das zweite Material **142** ist hier strangförmig innerhalb des ersten Materials **141** angeordnet und weist einen kreisförmigen, geschlossenen Querschnitt auf. Das zweite Material **142** ist vollständig von dem ersten Material **141** umgeben.

**Figur 10** zeigt das zweite Ende eines Schlauchs **103** mit einem in die Schlauchwand eingebetteten zweiten Material **142** als Querschnittsansicht, wobei das zweite Material **142** koaxial umlaufend in dem Schlauch angeordnet ist. Das zweite Material **142** weist hier einen kreisförmigen, offenen Querschnitt auf. Das zweite Material **142** bildet hier eine vollständig radial umlaufende Schicht, welche vollständig in das erste Material **141** eingebettet ist. Das erste Material **141** und das zweite Material **142** bilden gemeinsam eine monolithische Einheit, so dass das erste Material **141** und das zweite Material **142** nicht gegeneinander verschiebbar sind.

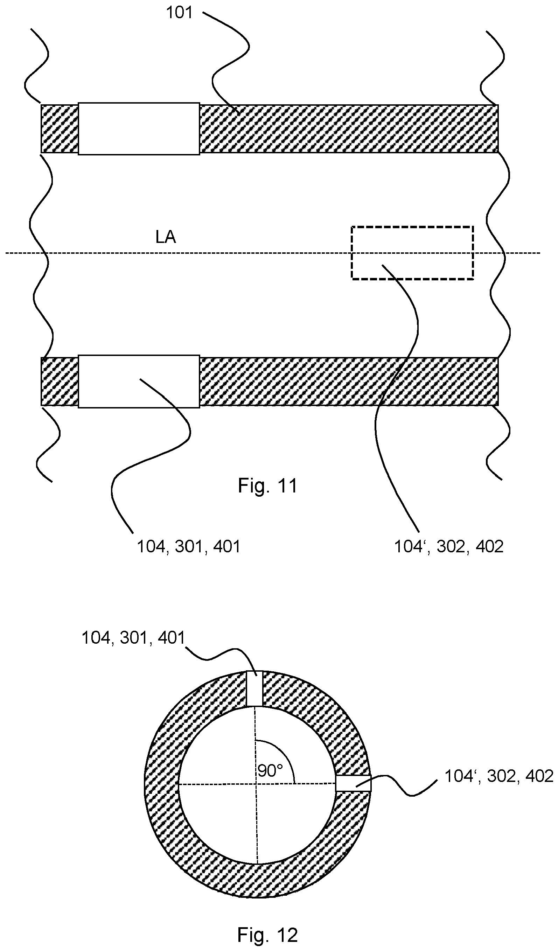

**Figur 11** zeigt einen Ausschnitt eines Schlauchs **101** in einer Längsschnittansicht, wobei zwei Schlitze **104, 104'** versetzt zueinander angeordnet sind. Ein erster Schlitz **104** ist an einer ersten longitudinalen Position **301** und an einer ersten radialen Position **401** des Schlauchs angeordnet. Ein zweiter Schlitz **104'** ist an einer zweiten longitudinalen Position **302** und ein einer zweiten radialen Position **402** des Schlauchs angeordnet. Hierbei unterscheidet sich jeweils die erste longitudinale Position von der zweiten longitudinalen Position, und die erste radiale Position von der zweiten radialen Position. Die beiden Schlitze **104**, **104'** sind in Längsrichtung des Schlauchs, d.h. parallel zu einer Längsachse **LA** des Schlauchs, zueinander versetzt und weisen dabei in unterschiedliche radiale Richtungen, um ein medizinisches Fluid an voneinander räumlich getrennte Zielbereiche abzugeben.

**Figur 12** zeigt einen Ausschnitt eines Schlauchs in einer Querschnittsansicht, wobei zwei Schlitze **104**, **104'** versetzt zueinander angeordnet sind. Ein erster Schlitz **104** ist an einer ersten longitudinalen Position **301** und an einer ersten radialen Position **401** des Schlauchs angeordnet. Ein zweiter Schlitz 104' ist an einer zweiten longitudinalen Position 302 und ein einer zweiten radialen Position 402 des Schlauchs angeordnet. Hierbei bildet die erste radiale Position 401 einen Winkel von etwa 90° zur zweiten radialen Position 402, wobei sich die Schenkel des Winkels in der Längsachse des Schlauchs schneiden.

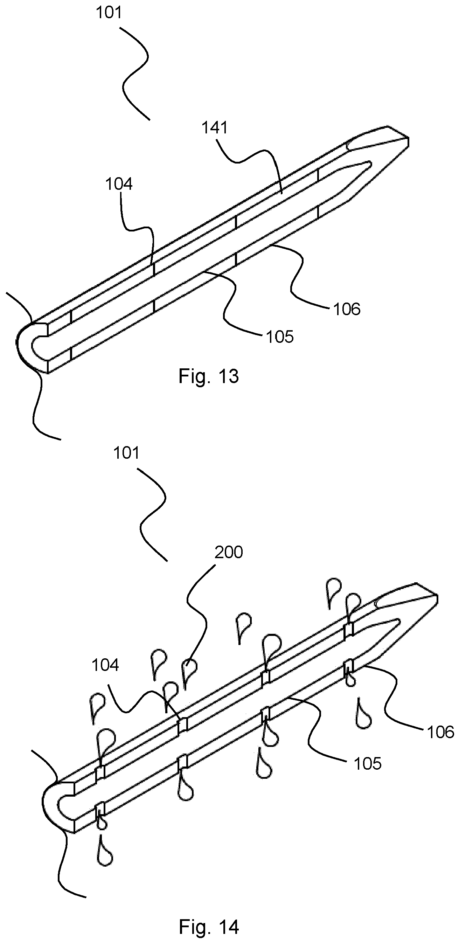

Figur 13 zeigt einen Ausschnitt eines Schlauchs 101 in einer Längsschnittansicht, wobei die Schlitze 104 in dem Schlauch geschlossen sind. Die Schlitze 104 erstrecken sich jeweils von der Innenseite 105 des Schlauchs zur Außenseite 106 des Schlauchs. Durch die Rückstellkraft des ersten Materials **141** werden die Schlitze **104** zusammengedrückt, und bleiben somit flüssigkeitsdicht geschlossen, solange keine Kraft auf den Schlauch einwirkt.

**Figur 14** zeigt einen Ausschnitt eines Schlauchs **101** in einer Längsschnittansicht, wobei die Schlitze **104** in dem Schlauch geöffnet sind. Hierbei drückt ein druckbeaufschlagtes medizinisches Fluid **200**, welches in dem Schlauch aufgenommen ist, gegen die Innenseite **105** des Schlauchs, sodass die Schlitze **104** auseinandergedrückt werden, und sich öffnen.

Hierdurch kann das medizinische Fluid **200** aus dem Lumen des Schlauchs durch die Schlitze **104** nach außen abgegeben werden. Der Öffnungszustand der Schlitze **104** kann hierbei durch den Druck des medizinischen Fluids gesteuert werden.

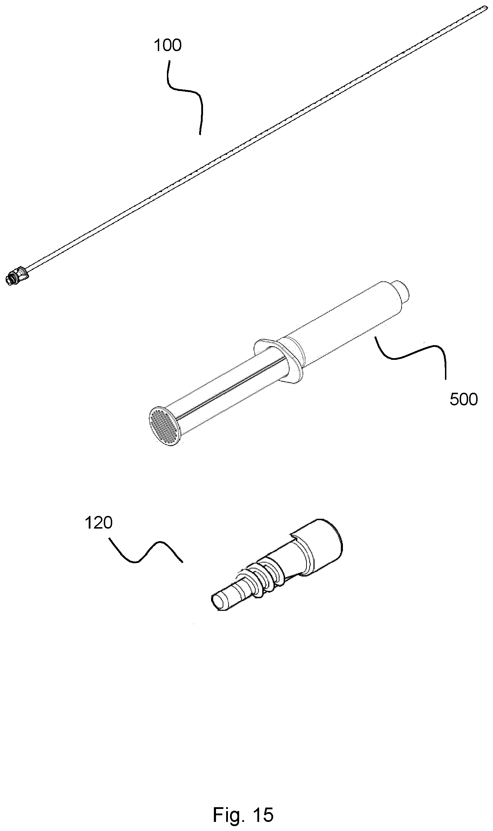

**Figur 15** zeigt ein Kit, welches eine hierin beschriebene Vorrichtung **100**, ein Mittel **500** zum Einbringen eines medizinischen Fluids, welches hier als Spritze ausgestaltet ist, und ein Mittel **120** zum Verschließen des zweiten Endes der Vorrichtung, welches hier als Stopfen ausgestaltet ist, enthält. Mithilfe der Spritze **500** kann ein medizinisches Fluid in die Vorrichtung **100** eingebracht werden, insbesondere durch Verbindung der Spritze **500** mit dem ersten Ende des Schlauchs der Vorrichtung **100.** Der Stopfen **120** kann verwendet werden, um das zweite Ende des Schlauchs der Vorrichtung **100** flüssigkeitsdicht zu verschließen. Gegebenenfalls kann ein Anwender die Länge des Schlauchs kürzen, bevor er den Schlauch mit dem Stopfen **120** am zweiten Ende des Schlauchs der Vorrichtung verschließt.

### BEZUGSZEICHENLISTE

- 100: Vorrichtung
- 101: Schlauch
- 102: erstes Ende
- 103: zweites Ende
- 104: Schlitz
- 105: Innenseite des Schlauchs
- 106: Außenseite des Schlauchs
- 112: erster Schlauchbereich
- 113: zweiter Schlauchbereich
- 120: Mittel zum Verschließen des zweiten Endes
- 130: Stützstruktur
- 141: erstes Material
- 142: zweites Material
- 200: Fluid
- 212: Fluidverbinder
- 220: Luer-Lock-Anschluss
- 230: Schlauchverbinder
- 240: Rückschlagventil
- 301: erste longitudinale Position
- 302: zweite longitudinale Position
- 401: erste radiale Position
- 402: zweite radiale Position
- 500: Mittel zum Einbringen eines medizinischen Fluids
- A: Abstand zwischen zwei Schlitzen an der Außenseite des Schlauchs
- AD: Außendurchmesser des Schlauchs
- L: Schlitzlänge
- LA: Längsachse des Schlauchs
- ID: Innendurchmesser des Schlauchs

## Patentansprüche

1. Vorrichtung (100) zur Abgabe eines medizinischen Fluids an einen Patienten, aufweisend einen Schlauch (101), welcher ein erstes medizinisch verträgliches Material (141) mit einer Shore-A-Härte im Bereich von 75 bis 95 aufweist,
wobei der Schlauch (101) einen Innendurchmesser (ID) und einen Außendurchmesser (AD) aufweist,
wobei das Verhältnis von [ID : AD] im Bereich von [1 : 1,8] bis [1 : 2,5] liegt;
wobei der Schlauch (101) ein erstes Ende (102) zur Aufnahme eines Fluids (200) in den Schlauch (101) aufweist;
wobei der Schlauch (101) ein zweites Ende (103) aufweist, das eingerichtet ist, ein Fluid (200) in dem Schlauch (101) zurückzuhalten;
ein oder mehrere Schlitze (104) zur Abgabe eines Fluids (200) aus dem Schlauch (101), wobei die Schlitze (104) jeweils einen Durchgang bilden, der sich von einer Innenseite (105) des Schlauchs (101) zu einer Außenseite (106) des Schlauchs erstreckt;
und wobei die Schlitze (104) eingerichtet sind, sich abhängig vom Druck eines Fluids (200) innerhalb des Schlauchs (101) reversibel zu öffnen, so dass das Fluid (200) aus den Schlitzen (104) abgegeben wird.

2. Vorrichtung nach Anspruch 1, wobei die Vorrichtung ausgebildet und eingerichtet ist, ein medizinisches Fluid ausschließlich über die Schlitze abzugeben, wobei bevorzugt eine gleichzeitige Abgabe des Fluids über mehrere Schlitze an unterschiedlichen Positionen entlang der Längsachse (LA) des Schlauchs erfolgt.

3. Vorrichtung nach einem der vorangehenden Ansprüche, wobei die Schlitze ausgebildet und eingerichtet sind, sich oberhalb eines Grenzdrucks eines Fluids innerhalb des Schlauchs zu öffnen, welcher 1 bar (10^5 Pa), bevorzugt 1,4 bar, höher als der Druck außerhalb des Schlauchs beträgt, und sich unterhalb dieses Grenzdrucks fluiddicht zu schließen.

4. Vorrichtung nach einem der vorangehenden Ansprüche, wobei die Schlitze eingerichtet sind, sich durch eine elastische Rückstellkraft des ersten Materials des Schlauchs reversibel zu öffnen und schließen.

5. Vorrichtung nach einem der vorangehenden Ansprüche, wobei die Schlitze jeweils eine Schlitzlänge (L) aufweisen, und wobei das Verhältnis der Schlitzlänge (L) zum Innendurchmesser (ID) des Schlauchs [L:ID] im Bereich von [1 : 2,2] bis [1 : 2,9] liegt, und/oder das Verhältnis der Schlitzlänge L zum Außendurchmesser (AD) des Schlauchs [L : AD] im Bereich von [1 : 3,5] bis [1 : 5,5] liegt.

6. Vorrichtung nach einem der vorangehenden Ansprüche, wobei die Vorrichtung ausgebildet und eingerichtet ist, den Außendurchmesser (AD) des Schlauchs um weniger als 10% zu verändern, wenn der Druck eines Fluids innerhalb des Schlauchs von 0 auf 1 bar (10^5 Pa) höher als der Druck außerhalb des Schlauchs steigt.

7. Vorrichtung nach einem der vorangehenden Ansprüche, wobei die Vorrichtung ausgebildet und eingerichtet ist, die Länge des Schlauchs um weniger als 10%, bevorzugt weniger als 5%, zu verändern, wenn der Druck eines Fluids innerhalb des Schlauchs von 0 auf 1 bar (10^5 Pa) höher als der Druck außerhalb des Schlauchs steigt.

8. Vorrichtung nach einem der vorangehenden Ansprüche, wobei der Schlauch mehrere Schlitze aufweist, die jeweils in einem Abstand (A) an der Außenseite des Schlauchs zueinander angeordnet sind, und jeweils eine Schlitzlänge (L) entlang der Außenseite des Schlauchs aufweisen, wobei das Verhältnis von Abstand (A) zur Schlitzlänge (L) [A : L] mindestens [10 : 1] beträgt.

9. Vorrichtung nach einem der vorangehenden Ansprüche, wobei der Schlauch ein zweites Material (142) aufweist, welches eine höhere Shore-A-Härte als das erste Material (141) aufweist, wobei das zweite Material bevorzugt als koaxial umlaufende Schicht oder als Streifen parallel zu einer Längsachse (LA) des Schlauchs angeordnet ist, wobei das zweite Material weiter bevorzugt ein Elastomer aufweist, und wobei weiterhin bevorzugt das zweite Material in das erste Material vollständig eingebettet ist.

10. Vorrichtung nach Anspruch 9, wobei das zweite Material einen Röntgenopaker und/oder einen Farbstoff aufweist, wobei der Röntgenopaker bevorzugt Bariumsulfat oder Wolfram umfasst, und wobei der Farbstoff bevorzugt ein absolutes Emissionsmaximum im Bereich von 490 nm bis 575 nm aufweist.

11. Vorrichtung nach einem der vorangehenden Ansprüche, wobei die Schlitze parallel zu einer Längsachse (LA) des Schlauchs angeordnet sind.

12. Vorrichtung nach einem der vorangehenden Ansprüche, wobei in einer ersten longitudinalen Position (301) in Richtung einer Längsachse (LA) des Schlauchs ein erster Schlitz (104) an einer ersten radialen Position (401) angeordnet ist, und in einer zweiten longitudinalen Position (302) in Richtung der Längsachse des Schlauchs ein zweiter Schlitz (104') an einer zweiten radialen Position (402) angeordnet ist, wobei die erste radiale Position (401) einen Winkel von etwa 90° zur zweiten radialen Position (402) bildet.

13. Vorrichtung nach einem der vorangehenden Ansprüche, wobei der Schlauch eine Stützstruktur (130) aus Metall aufweist, wobei die Stützstruktur (130) bevorzugt eine Metallbeschichtung, eine Metallfolie, eine Metallspirale oder einen Metallfaden aufweist, und wobei die Stützstruktur bevorzugt in das erste Material (141) eingebettet oder an der Innenseite (105) des Schlauchs angeordnet ist.

14. Vorrichtung nach einem der vorangehenden Ansprüche, wobei das erste medizinisch verträgliche Material (141) ein Polymer aufweist, wobei das Polymer bevorzugt Polyetherurethan oder Ethylen-Propylen-dien-Kautschuk aufweist.

15. Vorrichtung nach einem der vorangehenden Ansprüche, weiterhin aufweisend einen Fluidverbinder (212), der an dem ersten Ende (102) des Schlauchs (101) angeordnet ist, bevorzugt lösbar angeordnet ist.

16. Vorrichtung nach Anspruch 15, wobei der Fluidverbinder einen Luer-Lock-Anschluss (220) und/oder ein Rückschlagventil (240) aufweist.

17. Vorrichtung nach einem der vorangehenden Ansprüche, wobei das zweite Ende (103) fluiddicht verschlossen oder verschließbar ist, bevorzugt durch Verschweißen, Verschmelzen oder mittels eines Stopfens oder Schraubverschlusses verschlossen oder verschließbar ist.

18. Vorrichtung nach einem der vorangehenden Ansprüche, wobei die Schlitze (104) durch abschnittsweise Auftrennung des Schlauchs herstellbar oder hergestellt sind, ohne dabei Material aus dem Schlauch zu entfernen.

19. Vorrichtung nach einem der vorangehenden Ansprüche, wobei die Vorrichtung ausgebildet und eingerichtet ist, die Länge des Schlauchs (101) dauerhaft zu verändern, beispielsweise durch Entfernen eines Schlauchabschnitts am zweiten Ende (103) des Schlauchs.

20. Vorrichtung nach einem der vorangehenden Ansprüche, wobei der Schlauch einstückig ausgebildet ist, und wobei die Vorrichtung keinen zweiten Schlauch aufweist.

21. Kit, aufweisend eine Vorrichtung nach einem der vorangehenden Ansprüche und ein Mittel (500) zum Einbringen eines medizinischen Fluids, welches mit dem ersten Ende (102) des Schlauchs verbindbar ist.

22. Kit nach Anspruch 21, weiterhin aufweisend ein Mittel (120) zum Verschließen des zweiten Endes (103) des Schlauchs.

23. Kit nach Anspruch 21 oder 22, weiterhin aufweisend ein medizinisches Fluid, welches einen Wirkstoff aufweist, wobei der Wirkstoff bevorzugt ausgewählt ist aus der Gruppe bestehend aus einem Antibiotikum, einem Antimykotikum, einem Antitumorwirkstoff, einem osteoinduktiven Wirkstoff, und einem entzündungshemmenden Wirkstoff.

24. Kit nach einem der Ansprüche 21 bis 23, weiterhin aufweisend einen Trokar, wobei der Trokar mit dem ersten Ende (102) des Schlauchs verbindbar ist, bevorzugt lösbar verbindbar ist.

25. Medizinisches Therapieverfahren, wobei das Therapieverfahren die Verabreichung eines medizinischen Fluids an einen Patienten mithilfe einer Vorrichtung nach einem der Ansprüche 1 bis 20 oder eines Kits nach einem der Ansprüche 21 bis 24 aufweist.

26. Medizinischer Wirkstoff zur Anwendung bei einer Knochenoperation, wobei der Wirkstoff ausgewählt ist aus der Gruppe bestehend aus einem Antibiotikum, einem Antimykotikum, einem Antitumorwirkstoff, einem osteoinduktiven Wirkstoff, und einem entzündungshemmenden Wirkstoff, wobei das Verfahren das Inkontaktbringen einer Vorrichtung nach einem der Ansprüche 1 bis 20 oder eines Kits nach einem der Ansprüche 21 bis 24 mit einer Operationswunde eines Patienten und die lokale Verabreichung des Wirkstoffs als medizinisches Fluid an die Operationswunde mithilfe der Vorrichtung oder des Kits aufweist.

27. Medizinischer Wirkstoff zur Therapie oder Prophylaxe einer Knochenerkrankung, wobei der Wirkstoff ausgewählt ist aus der Gruppe bestehend aus einem Antibiotikum, einem Antimykotikum, einem Antitumorwirkstoff, einem osteoinduktiven Wirkstoff, und einem entzündungshemmenden Wirkstoff, wobei das Verfahren die lokale Verabreichung des Wirkstoffs mithilfe einer hierin beschriebenen Vorrichtung nach einem der Ansprüche 1 bis 20 oder eines Kits nach einem der Ansprüche 21 bis 24 als medizinisches Fluid an ein Zielgewebe eines Patienten umfasst.
